(19) Europäisches Patentamt
European Patent Office
Office européen des brevets

(11) Veröffentlichungsnummer: **0 078 478**
**B1**

# (12) EUROPÄISCHE PATENTSCHRIFT

(45) Veröffentlichungstag der Patentschrift: **21.05.86**

(21) Anmeldenummer: **82109833.2**

(22) Anmeldetag: **25.10.82**

(51) Int. Cl.4: **C 08 K 5/51,** C 08 L 75/04 // C07F9/11, C07F9/40, C07C103/44, C07C87/10, C07C91/06, C07C87/14, C07D295/12, C07C93/04, C07C87/20

(54) **Hochfrequenzverschweissbare Polyurethanschaumstoffe und ein Verfahren zur Herstellung derselben.**

(30) Priorität: **04.11.81 DE 3143706**

(43) Veröffentlichungstag der Anmeldung: **11.05.83 Patentblatt 83/19**

(45) Bekanntmachung des Hinweises auf die Patenterteilung: **21.05.86 Patentblatt 86/21**

(84) Benannte Vertragsstaaten: **BE DE FR GB IT NL**

(56) Entgegenhaltungen:
**EP - A - 0 025 572**
**AT - A - 328 749**
**FR - A - 2 239 489**
**FR - A - 2 374 379**

**CHEMICAL ABSTRACTS, Band 77, Nr. 8, 21. August 1972, Seite 55, Nr. 49530b, Columbus, Ohio, USA**

(73) Patentinhaber: **BAYER AG, Konzernverwaltung RP Patentabteilung, D-5090 Leverkusen 1 Bayerwerk (DE)**

(72) Erfinder: **Seifert, Peter, Dr., August-Kierspel-Strasse 161, D-5060 Bergisch-Gladbach 2 (DE)**
Erfinder: **Haas, Peter, Dr., Zwengenberger Strasse 43, D-5657 Haan 1 (DE)**

**EP 0 078 478 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Gegenstand der Erfindung sind hochfrequenzverschweißbare Polyurethanschaumstoffe und ein Verfahren zur Herstellung derselben, gekennzeichnet durch den Zusatz von bestimmten Ammoniumsalzen phosphorhaltiger Säuren zu den Polyurethanschaumstoff-bildenden Ausgangskomponenten, so daß der Gehalt an Ammoniumsalzen der Phosphorsäuren 0,1 bis 10 Gew.-% in den Polyurethanausgangsstoffen bzw. Polyurethanen beträgt.

Für die Hochfrequenzverschweißbarkeit von Kunststoffmaterialien gibt es zwei Kriterien:

1. Thermoplastizität: Dies bedeutet, daß das Material bei einer Temperatur unterhalb seiner Zersetzungstemperatur vom festen in den flüssigen Zustand übergehen muß, evtl. und vorzugsweise über einen vorgelagerten Erweichungsbereich.
2. Dielektrischer Verlustfaktor: Der dielektrische Verlustfaktor beinhaltet die Aufnahmefähigkeit für Energie aus einem hochfrequenten elektrischen Wechselfeld. Diese Eigenschaft ist eine Materialkonstante.

Geschäumte Materialien werden in der Regel nicht mit sich selbst, sondern mit textilen bzw. folienartigen Materialien oder mit Preßpappe verschweißt. Der dielektrische Verlustfaktor das Gesamtverbundes wird naturgemäß von diesen Partnermaterialien stark beeinflußt. Häufig überspielt der Effekt der Partnermaterialien den des Polyurethanschaumes. Dennoch ist ein hoher dielektrischer Verlustfaktor auch bei den Schaumstoffen erstrebenswert, da er die Verschweißung mit beliebigen Materialien möglich macht, also auch mit solchen, die selbst keinen dielektrischen Verlustfaktor besitzen (z.B. Textilien aus reinen Polyethylenterephthalatfasern).

Von den gebräuchlichen, homogen geschäumten Materialien erfüllt bisher nur PVC-Schaum sowohl die Bedingungen der Thermoplastizität als auch die des dielektrischen Verlustfaktors in weitem Maße. Sein hoher Preis, sein hohes Gewicht sowie seine schlechten mechanischen Eigenschaften stehen seinem breiten Einsatz jedoch im Wege.

An Weiteren HF-schweißfähigen Materialien stehen thermoplastmodifizierte Polyurethan-Schaumstoffe auf Polyesterbasis zur Verfügung.

Verfahren, Polyurethanschaum HF-schweißfähig zu machen, sind das Benadeln mit thermoplastischen Fasern mit dielektrischem Verlustfaktor oder das Bestreuen mit thermoplastischen, HF-aktiven Pulvern.

Alle diese Verfahren haben systembedingte Nachteile. So muß beim Benadeln der Schaum durchstochen werden, weiterhin läßt sich die benadelte Oberfläche nicht mehr mit Deckschichten flammkaschieren. Beim Bestreuen mit Pulvern muß mit einem Herausrieseln des Pulvers gerechnet werden, was die Verarbeitungsmaschinen verschmutzen kann oder später zu Verschmutzungen der Polster führt. Beide dieser Verfahren sind naturgemäß teuer.

Besser funktioniert die Einarbeitung von Thermoplastpulvern in den Rohstoffstrom während des Schaumvorganges. Hier sind jedoch größeren Zusatzmengen durch den starken Viskositätsanstieg des ohnehin schon viskosen Polyesterpolyols Grenzen gesetzt. Weiterhin macht die gleichmäßige Verteilung der Feststoffe im Schaum sowie die unvermeidliche Gasbeladung Schwierigkeiten.

Ein großer Fortschritt auf dem Weg zu hochfrequenzverschweißbaren Schaumstoffen war die Entwicklung von Polyurethan-Lösungen in Polyetherpolyolen wie in DE-A 3 008 590 und auch DE-A 2 937 509 beschrieben. Solche Schaumstoffe lassen sich völlig homogen herstellen und sind mit allen HF-aktiven Materialien hervorragend schweißbar. Probleme treten jedoch noch bei der Verschweißung mit Deckschichten ohne dielektrischen Verlustfaktor auf (z.B. bei Textilien aus reinen Polyesterfasern), da der Schaumstoff aufgrund seines relativ geringen dielektrischen Verlustfaktors allein zu wenig Energie aufnimmt. Die Folge davon ist eine stark verlängerte Schweißzeit oder ein sehr hoher Energiebedarf, der die Gefahr von Durchbrennen erhöht. Zwar werden bei der Herstellung von HF-verschweißbaren Polyetherpolyurethanschaumstoffen nach der Lehre der DE-A 3 008 590 bzw. 2 937 509 gegenüber den Standard-Polyetherpolyurethanschaumstoffen schon erhebliche Verbesserungen bewirkt, jedoch zeigt die Praxis, daß dies dennoch verbesserungsbedürftig ist. Ammoniumphosphate nach der FR-PS 2 374 379 sind hochschmelzende und in den Polyolen nicht lösliche Verbindungen, die sich nur sehr ungünstig verarbeiten lassen und auch PU-Schaumstoffe nicht hochfrequenz-verschweißbar machen.

Überraschenderweise hat sich nun gezeigt, daß sich der dielektrische Verlustfaktor von thermoplastischen PU-Schaumstoffen, auch der nach den deutschen Offenlegungsschriften 3 008 590 und 2 937 590 herstellbaren thermoplastischen Schaumstoffe, durch den Zusatz der erfindungsgemäßen Ammoniumsalze phosphorhaltiger Säuren signifikant erhöhen bzw. sehr deutlich weiter erhöhen läßt.

Das Ergebnis ist, wie die Beispiele im experimentellen Teil zeigen, eine erheblich verbesserte HF-Verschweißbarkeit, auch mit an sich nicht HF-erwärmbaren Materialien. Es können so z.B. auch Textilien nach dieser Technik problemfrei verarbeitet werden, die selbst keinen dielektrischen Verlustfaktor aufweisen, und wo dieser ganz vom Schaumstoff aufgebracht werden muß. Die Erhöhung des dielektrischen Verlustfaktors gelang nun überraschend dadurch, daß man Ammoniumsalze phosphorhaltiger Säuren in die Polyurethanschaumstoffe einbringt.

Gegenstand der Erfindung ist daher ein Verfahren zur Herstellung von leicht hochfrequenzverschweißbaren Polyurethanschaumstoffen durch Umsetzung von mindestens zwei gegenüber Isocyanaten reaktionsfähigen Wasserstoffatome aufweisenden höhermolekularen Verbindungen vom Molekulargewicht 400 bis 10 000 mit Polyisocyanaten und gegebenenfalls Kettenverlängerungsmitteln vom Molekulargewicht 18 bis 399 in Gegenwart von Katalysatoren, gegebenenfalls in Gegenwart von Schaumstabilisatoren, Wasser und/oder organischen Treibmittelzusätzen und üblichen Hilfs- und Zusatzstoffen, dadurch gekennzeichnet, daß man Ammoniumsalze

phosphorhaltiger Säuren einsetz, die glatt in Polyolen löslich sind und Phosphorsäure-mono- und -dialkylester, Dialkyl-phosphinsäuren, Alkylphosphonsäuren bzw. ihre Monoalkylester als phosphorhaltige Säuren enthalten, und sie den Schaumstoff-Ausgangskomponenten, gelöst in einer oder mehreren der Ausgangskomponenten, in Mengen von 0,1 bis 10 Gew.-%, bezogen auf die Gesamtmischung, zusetzt. Bevorzugt sind Salze der Formel

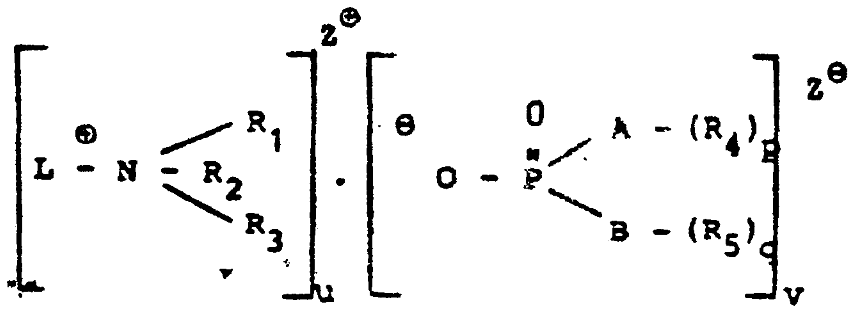

[Kation] . [Anion]

( es gilt: $u \cdot z^{\oplus} = v \cdot z^{\ominus}$ )

wobei

I. für den kationischen Aminteil

| | |
|---|---|
| L | Wasserstoff und/oder Alkyl von $C_1$ bis $C_6$ |
| $R_{1,2,3}$ | $—(CH_2)_e—T$, wobei |
| e | eine ganze Zahl von 1 bis 10 und |
| T | Wasserstoff, Hydroxy, 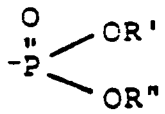 und $—O—(CH_2)_e$ 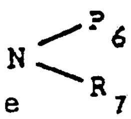 |
| | und |
| R', R" | gleichen oder verschiedenen Alkylresten von $C_1$ bis $C_{10}$, gegebenenfalls auch durch Hydroxy substituiert, oder |
| | NY—C—R' ", mit |
| R' " | gleich Wasserstoff oder ein Alkylrest von $C_1$ bis $C_6$; |
| Y | Wasserstoff oder $—(CH_2)_e—T$ sein kann und/oder |
| $R_{1,2,3}$ | $—(CH_2)_n—N$ 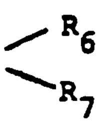 wobei |
| $R_6$, $R_7$ | gleiche oder verschiedene, lineare oder verzweigte Alkylreste von $C_1$ bis $C_6$, bevorzugt Methyl oder Ethyl, |
| n | eine ganze Zahl von 2 bis 10 und/oder |

$R_{1,2,3}$ 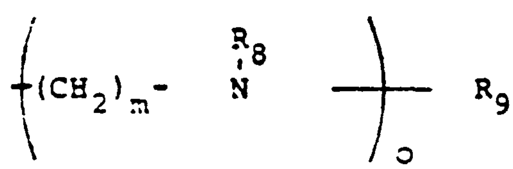

wobei

| | |
|---|---|
| $R_8$, $R_9$ | gleiche oder verschiedene lineare oder verzweigte Alkylreste von $C_1$ bis $C_6$, bevorzugt Methyl oder Ethyl, |
| m | eine ganze Zahl von 2 bis 10, |
| o | eine ganze Zahl von 2 bis 6 sein kann und |
| $Z^{\oplus}$ | die Ladungzahl, des Kations vorzugsweise 1 oder 2, ist und der Gesamtzahl an N-Atomen entspricht oder kleiner als diese sein kann, |
| u | je nach Ladungszahl $Z^{\oplus}$ des Kationenrestes dieser u-mal (vorzugsweise 1- oder 2-mal) enthalten sein kann, |
| | wobei gilt, daß $u \cdot Z^{\oplus} = v \cdot Z^{\ominus}$ ist und das Produkt vorzugsweise 1 oder 2 bedeutet; |

II. sowie für das phosphorhaltige Anion

A, —$CH_2$— oder —O—

B, —$CH_2$—

$Z^{\ominus}$ die Ladungszahl des Anions ist,

v je nach Ladungszahl $Z^{\ominus}$ das Anions dieser anionische Rest v-mal enthalten ist, und eine ganze Zahl zwischen 1 und 3 bedeutet, wofür gilt, daß $v \cdot Z^{\ominus} = u \cdot Z^{\oplus}$ ist,

$R_4$, $R_5$ Wasserstoff und/oder einen $C_1$—$C_{10}$—Alkyl— oder -Cycloalkylrest.

den Schaumstoff-Ausgangskomponenten, gelöst in einer oder mehrerer der Ausgangskomponenten in Mengen von 0,1 bis 10 Gew.-%, bezogen auf die Gesamtmischung, zusetzt. Bevorzugt wird ein Verfahren, bei dem man die Ammoniumsalze der phosphorhaltigen Säuren in den höhermolekularen Verbindungen vom Molekulargewicht 400 bis 10 000 löst und damit die Schaumstoffbildung durchführt. Weniger bevorzugt ist es, die Ammoniumsalze in den Polyisocyanaten zu lösen und dann die Schaumstoffbildung durchzuführen.

Wenn z.B. das Kation einwertig ist (1 $N^{\oplus}$), so ist auch das Anion einwertig. Wenn das Kation zweiwertig ist (z.B. 2 $N^{\oplus}$), so muß das Anion auch zweiwertig sein (z.B. $A=O^{\ominus}$) oder es müssen 2 einwertige Anionen vorhanden sein; bei diesem Beispiel können dem zweiwertigen Anion jedoch auch 2 einwertige Kationen gegenü berstehen.

Gegenstand der Erfindung sind jedoch auch hochfrequenzverschweißbare Polyurethanschaumstoffe aus höhermolekularen Polyhydroxylverbindungen vom Molekulargewicht 400 bis 10 000. Polyisocyanaten, gegebenenfalls Kettenverlängerungsmitteln vom Molekulargewicht 18 bis 399, Katalysatoren, gegebenenfalls Schaumstabilisatoren, Wasser und/oder organischen Treibmittelzusätzen und Hilfs- und Zusatzstoffen, dadurch gekennzeishnet, daß sie

0,1 bis 10 Gew.-% Ammoniumsalze phosphorhaltiger Säuren der Formel

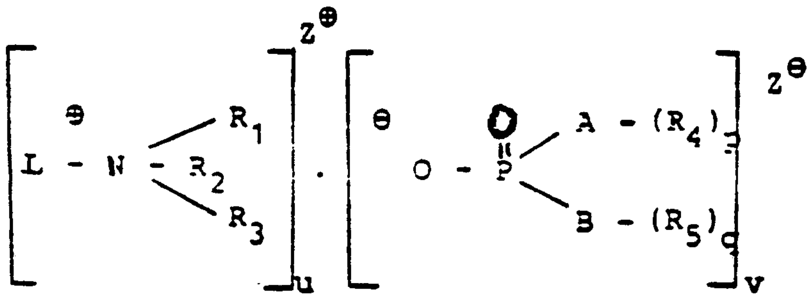

[Kation] [Anion]

(es gilt: $u \cdot Z^{\oplus} = \cdot Z^{\ominus}$)

enthalten, wobei die Substituenten am Stickstoff und Phosphor die vorher angegebene Bedeutung haben.

Die erfindungsgemäßen Zusätze zur Erhöhung der HF-Verschweißbarkeit von PU-Schaumstoffen, besonders polyetherhaltigen PU-Schaumstoffen, sind Ammoniumsalte phosphorhaltiger Säuren, die in flüssiger Form liegen und glatt in Polyolen löslich sind. Die phosphorhaltigen Säuren sind Phosphorsäure-mono- und -dialkylester, Dialkyl - phosphinsäuren, Alkylphosphonsäuren bzw. ihre Monoalkylester. Hier sind besonders die Salze des Phosphorsäuredibutylesters sowie des Phosphorsäure-di-(2-ethylhexyl)-esters bevorzugt, wobei vorzugsweise als Aminkomponente die in den DE-A 2 624 527, 2 732 292, 2 909 482, 3 027 796 und 3 046 905 beschriebenen geruchsarmen Amine zur Ammoniumsalzbildung herangezogen werden. Dies sind z.B.:

Als Beispiele für die erfindungsgemäße Verbindungsklasse zur Erhöhung des dielektrischen Verlustfaktors bei der Hochfrequenzverschweißung von Polyurethanschaumstoffen seien genannt:

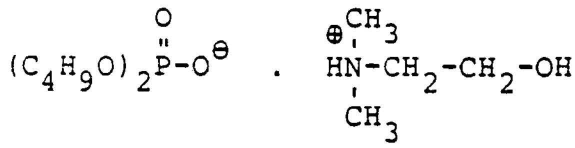

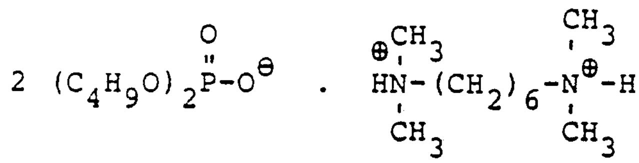

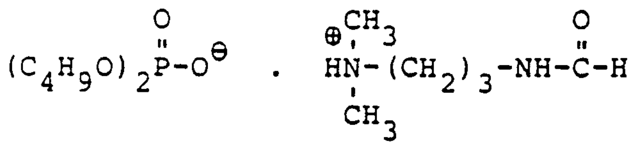

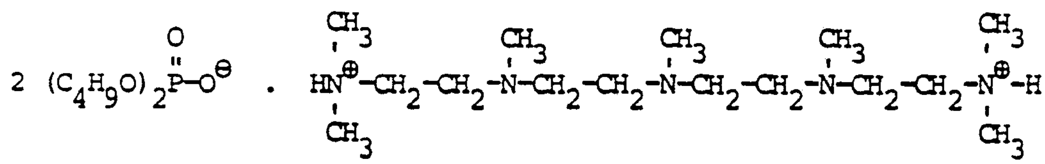

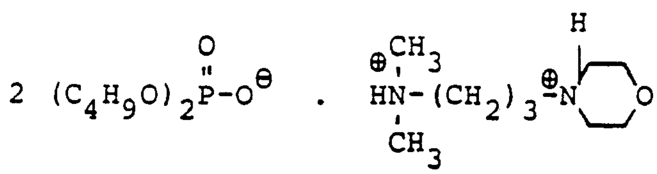

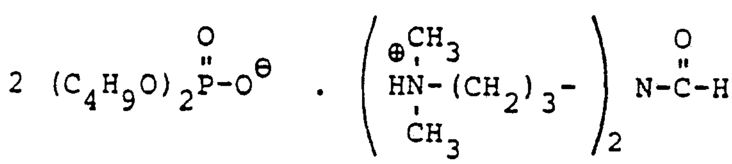

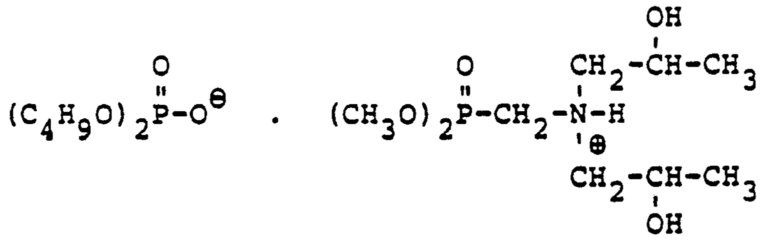

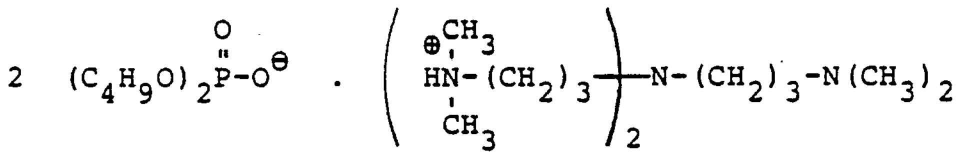

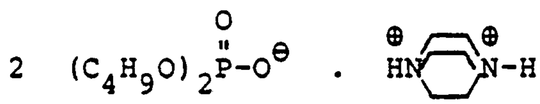

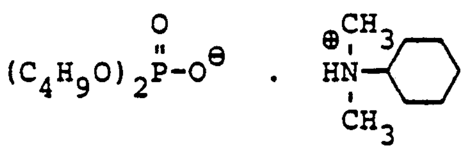

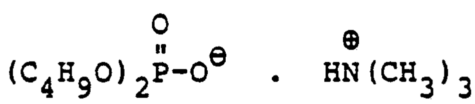

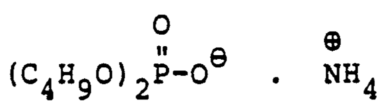

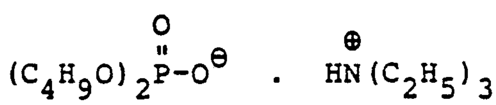

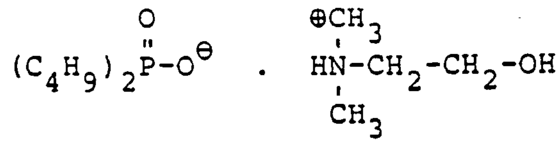

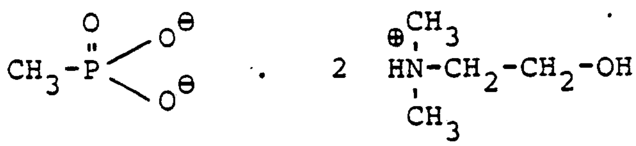

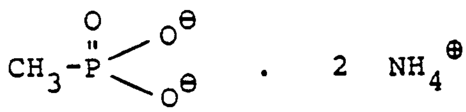

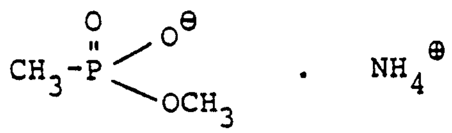

Zur Herstellung der erfindungsgemäßen Zusätze werden also phosphorhaltige Säuren, wie z.B. Phosphorsäuredibutylester, Phosphorsäure-bis-(2-ethyl-hexylester), Dibutylphosphinsäure, Methylphosphonsäure oder Methylphosphonsäuremonomethylester mit basischen Stickstoff enthaltenden Verbindungen wie Tetramethylhexamethylendiamin, Heptamethyltetraethylenpentamin, Dimethylaminopropylformamid, Bis-(dimethylaminopropyl)-formamid, Dimethylaminopropylmorpholin, Tris-(dimethylamino-n-propyl)-amin, Ethanolamin, Diethanolamin, Dimethyloethanolamin, Methyldiethanolamin, Triethylamin, Dimethylcyclohexylamin, Pentamethyldiethylentriamin, Dimethylaminopropyl-methyl-piperazin, Piperazin, Triethylendiamin, Diisopropylamin, Hexamethylendiamin, Bis-(2-hydroxyethyl)-amino-methylphosphonsäuredimethylester, Adipinsäure-bis-(2-dimethylaminoethylester), 1,8-Diazabicyclo-(5,4,0)-undec-7-en, 1,5-Diazabicyclo-(5,4,0)-non-5-en, Orthoameisensäure-tris-dimethylaminoethylester, Bis-(dimethylaminoethyl)-ether ganz oder teilweise neutralisiert.

Die ganz oder teilweise mit phosphorhaltigen Säuren protonierten Amine werden in Mengen von 0,1 bis 10 Teilen, vorzugsweise von 1 bis 5 Teilen, in der Polyurethan-Rezeptur verwendet, vorzugsweise in den Polyolstrom eingearbeitet. Es handelt sich bei den erfindungsgemäß einzusetzenden Produkten um größtenteils flüssige bis zähflüssige Körper, die gegebenenfalls zur besseren Handhabung in wäßriger Lösung oder in höherfunktionellen Alkoholen gelöst verarbeitet werden können.

Für Herstellung der Polyurethanschaumstoffe werden die an sich bekannten Ausgangskomponenten eingesetzt:

Dies sind höhermolekulare Verbindungen mit mindestens zwei gegenüber Isocyanaten reaktionsfähigen Wasserstoffatomen und einem Molekulargewicht von 400 bis 10 000. Hierunter versteht man neben Aminogruppen, Thiolgruppen oder Carboxylgruppen aufweisenden Verbindungen vorzugsweise solche mit Hydroxylgruppen, insbesondere 2 bis 8 Hydroxylgruppen und speziell solche mit einem Molekulargewicht von 1000 bis 6000, vorzugsweise 2000 bis 4000. Beispiele sind mindestens zwei, in der Regel zwei bis acht, vorzugsweise aber zwei bis vier, Hydroxylgruppen aufweisende Polyester, Polyether, Polythioether, Polyacetale, Polycarbonate, Polyactone und Polyesteramide, wie sie für die Herstellung von homogenen und zellförmigen Polyurethanen an sich bekannt sind und wie sie z.B. in der DE-A 2 832 253, Seiten 11—18, ausführlich beschrieben werden.

Zur Erzielung optimaler Resultate werden auch vorzugsweise die modifizierten Polyhydroxylverbindungen nach der Lehre der DE-A 3 008 590 und 2 937 509 eingesetzt.

Als Polyisocyanatkomponenten dienen aliphatische, cycloaliphatische, araliphatische, aromatische und heterocyclische Polyisocyanate, wie sie z.B. von W. Siefken in Justus Liebigs Annalen der Chemie, 362, Seiten 75—136, beschrieben werden, beispielsweise solche der Formel

$$Q(NCO)_n$$

in der

n 2 bis 4, vorzugsweise 2, und

Q einen aliphatischen Kohlenwasserstoffrest mit 2 bis 18, vorzugsweise 6 bis 10, C-Atomen, einen cycloaliphatischen Kohlenwasserstoffrest mit 4 bis 15, vorzugsweise 5 bis 10, C-Atomen, einen aromatischen Kohlenwasserstoffrest mit 6 bis 15, vorzugsweise 6 bis 13, C-Atomen, oder einen araliphatischen Kohlenwasserstoffrest mit 8 bis 15, vorzugsweise 8 bis 13, C-Atomen,

bedeuten, z.B. solche Polyisocyanate, wie sie in der DE-A 2 832 253, Seiten 10—11, beschrieben werden.

Besonders bevorzugt werden in der Regel die technisch leicht zugänglichen Polyisocyanate, z.B. das 2,4- und 2,6-Toluylendiisocyanat sowie beliebige Gemische dieser Isomeren ("TDI"), Polyphenyl-polymethylen-polyisocyanate, wie sie durch Anilin-Formaldehyd-Kondensation und anschließende Phosgenierung hergestellt werden ("rohes MDI") und Carbodiimidgruppen, Urethangruppen, Allphanatgruppen, Isocyanuratgruppen, Harnstoffgruppen oder Biuretgruppen aufweisende Polyisocyanate ("modifizierte Polyisocyanate"), insbesondere solche modifizierten Polyisocyanate, die sich vom 2,4- und/oder 2,6-Toluylendiisocyanat bzw. vom 4,4'-und/oder 2,4'-Diphenylmethandiisocyanat ableiten.

Als Ausgangskomponenten können gegebenenfalls Kettenverlängerungsmittel und/oder Vernetzer mit mindestens zwei gegenüber Isocyanaten reaktionsfähigen Wasserstoffatomen und Molekulargewichten von 32 bis 399 mitverwendet werden. Auch in diesem Fall versteht man hierunter Hydroxylgruppen und/oder Aminogruppen und/oder Thiolgruppen und/oder Carboxylgruppen aufweisende Verbindungen, vorzugsweise Hydroxylgruppen und/oder Aminogruppen aufweisende Verbindungen, die als Kettenverlängerungsmittel oder Vernetzungsmittel dienen. Diese Verbindungen weisen in der Regel 2 bis 8, vorzugsweise 2 bis 4, gegenüber Isocyanaten reaktionsfähige Wasserstoffatome auf. Beispiele hierfür werden in der DE-A 2 832 253, Seite 19—20, beschreieben.

Gegebenenfalls können als Hilfs- und Zusatzmittel Substanzen wie

a) Wasser und/oder leicht flüchtige anorganische oder organische Substanzen als Treibmittel,
b) Katalysatoren der an sich bekannten Art,
c) oberflächenaktive Zusatzstoffe, wie Emulgatoren und Schaumstabilisatoren,
d) Reaktionsverzögerer, z.B. sauer reagierende Stoffe wie Salzsäure oder organische Säurehalogenide, ferner Zellregler der an sich bekannten Art wie Paraffine oder Fettalkohole oder Dimethylpolysiloxane sowie Pigmente oder Farbstoffe und Flammschutzmittel der an sich bekannten Art, z.B. Trischlorethylphosphat, Trikresylphosphat oder Ammonioumphosphat und -polyphosphat, ferner Stabilisatoren gegen Alterungs- und Witterungseinflüsse, Weichmacher und fungistatisch und bakteriostatisch wirkende Substanzen sowie Füllstoffe wie Bariumsulfat, Kieselgur, Ruß oder Schlämmkreide verwendet werden..

Diese gegebenenfalls mitzuverwendenden Hilfs- und Zusatzstoffe wurden beispielsweise in der DE-A 2 732 292, Seiten 21—24, beschrieben.

Weitere Beispiele von gegebenenfalls erfindungsgemäß mitzuverwendenden oberflächenaktiven Zusatzstoffen und Schaumstabilisatoren sowie Zellreglern, Reaktionsverzögerern, Stabilisatoren, flammhemmende Substanzen, Weichmachern, Farbstoffen und Füllstoffen sowie fungistatisch und bakteriostatisch wirksamen Sbustanzen sowie Einzelheiten über Verwendungs- und Wirkungsweise dieser Zusatzmittel sind im Kunststoff-Handbuch, Band VII, herausgegeben von Vieweg und Höchtlen, Carl-Hanser-Verlag, München 1966, z.B. auf den Seiten 103 bis 113 beschrieben.

Zur Durchführung des erfindungsgemäßen Verfahrens:

Die Reaktionskomponenten, von denen eine oder mehrere die Ammoniumsalze der Phosphorsäuren enthalten, werden nach dem an sich bekannten Einstufenverfahren, dem Prepolymerverfahren oder dem Semiprepolymerverfahren zur Umsetzung gebracht, wobei man sich oft maschineller Einrichtungen bedient, z.B. solcher, die in der US-Patentschrift 2 764 565 beschreiben werden. Einzelheiten über Verarbeitungseinrichtungen, die auch erfindungsgemäß in Frage kommen, werden im Kunststoff-Handbuch, Band VII, herausgegeben von Vieweg und Höchtlen, Carl-Hanser-Verlag, München 1966, z.B. auf den Seiten 121—205, beschrieben.

Bei der Schaumstoffherstellung wird erfindungsgemäß die Verschäumung bevorzugt in geschlossenen Formen durchgeführt. Dabei wird das Reaktionsgemisch in eine Form eingetragen. Als Formmaterial kommt Metall, z.B. Aluminium oder Kunststoff, z.B. Epoxidharz, in Frage. In der Form schäumt das schäumfähige Reaktionsgemisch auf und bildet den Formkörper. Die Formverschäumung kann dabei so durchgeführt werden, daß das Formteil an seiner Oberfläche Zellstruktur aufweist, sie kann aber auch so durchgeführt werden, daß das Formteil eine kompakte Haut und einen zelligen Kern aufweist. Erfindungsgemäß kann mann in diesem Zusammenhang so vorgehen, daß man in die Form so viel schäumfähiges Reaktionsgemisch einträgt, daß der gebildete Schaumstoff die Form gerade ausfüllt. Man kann aber auch so arbeiten, daß man mehr schäumfähiges Reaktionsgemisch in die Form einträgt, als zur Ausfüllung des Fornminneren mit Schaumstoff notwendig ist. Im letztgenannten Fall wird somit unter "overcharging" gearbeitet; eine derartige Verfahrensweise ist z.B. aus den US-Patentschriften 3 178 490 und 3 182 104 bekannt.

Bei der bevorzugten Formverschäumung werden vielfach an sich bekannte "äußere Trennmittel", Wie Siliconöle,

mitverwendet. Man kann aber auch sogenannte "innere Trennmittel", gegebenenfalls im Gemisch mit äußeren Trennmitteln, verwenden, wie sie z.B. aus den DE-A 2 121 670 und 2 307 589 bekanntgeworden sind.

Erfindungsgemäß ist bevorzugt, in kalter Form geschäumte kalthärtende Formschaumstoffe herzustellen (vgl. GB-PS 1 162 517, DE-A 2 153 086).

Selbstverständlich können aber auch Schaumstoffe durch Blockverschäumung oder nach dem an sich bekannten Doppeltransportbandverfahren hergestellt werden.

Einsatzgebiete für Polyurethanschaumstoffe, vorzugsweise Polyetherurethanschaumstoffe, welche durch die erfindungsgemäßen Additive einen erhöhten dielektrischen Verlustfaktor aufweisen, sind beispielsweise: textil- oder folienverkleidete Polyurethanauflagen für Autositze, Türverkleidungen, Griffe, Autoinnenausstattungen, sowie diverse Sandwich- oder Schichtmaterialien, die in der Täschnerindustrie, Schuhindustrie, Möbelindustrie etc. Anwendung finden können.

**Experimenteller Teil**

A) Herstellung der Ammoniumsalze phosphorhaltiger Säuren:

1.

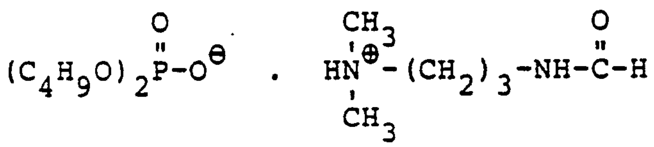

In 210 g (1 mol) Phosphorsäuredibutylester werden bei 10°C unter Kühlung 130 g (1 mol) Dimethylaminpropylformamid getropft; das viskose Reaktionsprodukt vom $n_D^{20}$=1,4615 kristallisiert langsam, hat einen Schmelzpunkt von 28°C und kann in hohen Konzentrationen in wäßriger Lösung flüssig gehalten werden, $n_D^{20}$=1,4428 (80 %ige Lösung in Wasser);

2.

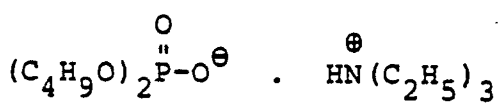

Nach Beispiel 1 aus 210 g (1 mol) Phosphorsäuredibutylester und 101 g (1 mol) Triethylamin; $n_D^{20}$=1,4440.

3.

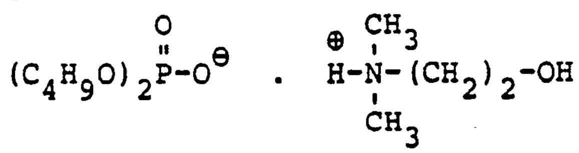

Nach Beispiel 1 aus 210 g (1 mol) Phosphorsäuredibutylester und 89 g (1 mol) Dimethylethanolamin; $n_D^{20}$=1,4750.

4.

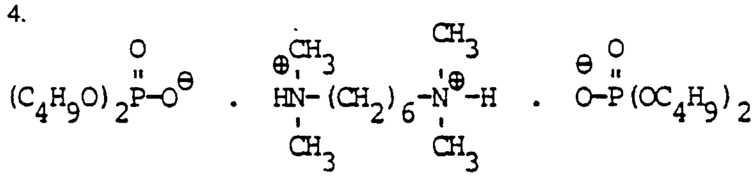

Nach Beispiel 1 aus 420 g (2 mol) Phosphorsäuredibutylester und 172 g (1 mol) Tetramethylhexamethylendiamin; $n_D^{20}$=1,4549.

5.

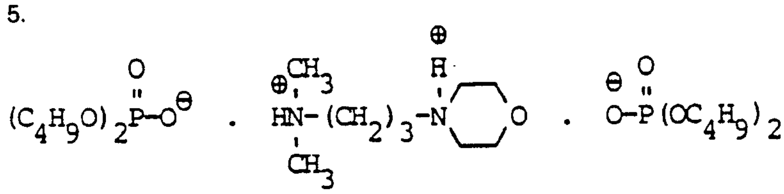

Nach Beispiel 1 aus 210 g (1 mol) Phosphorsäuredibutylester und 86 g (0,5 mol) Dimethylaminopropylmorpholin; $n_D^{20}$=1,4595.

6.

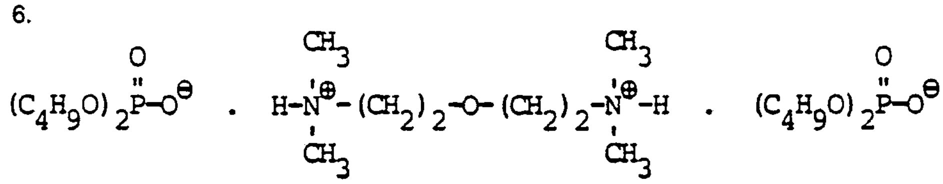

Nach Beispiel 1 aus 210 g (1 mol) Phosphorsäuredibutylester und 80 g (0,5 mol) Bis-(dimethylaminoethyl)-ether.

7.

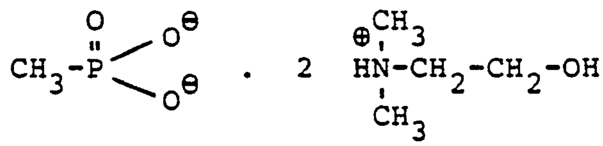

Zu 39 g (0,40 mol) Methylphosphonsäure in 100 ml Methanol werden 72,5 g (0,8 mol) Dimethylethanolamin unter Kühlung getropft und anschließend eingeengt. Quant. Ausbeute eines gelben Öls.

8.

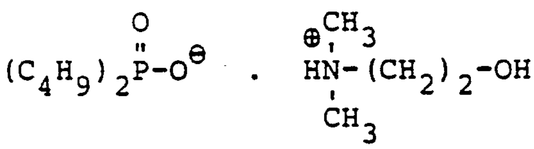

Zu 31 g (0,174 mol) Dibutylphosphinsäure werden 15,5 g (0,174 mol) Dimethylethanolamin getropft; quant. Ausbeute eines gelblichen Öls.

9.

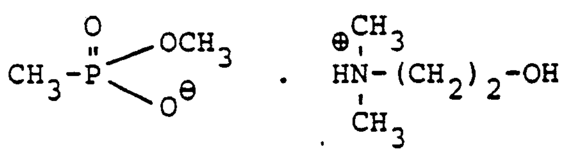

Zu 54 g (0,455 mol) Methylphosphonsäuremonomethylester werden 40 g (0,45 mol) Dimethylethanolamin getropft.

10.

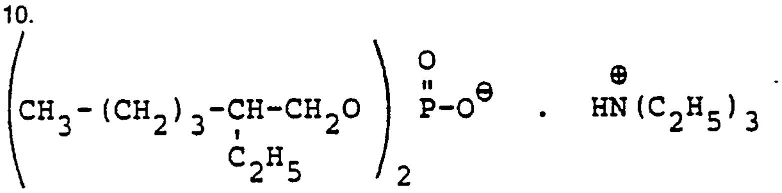

Aus 322 g (1 mol) Phosphorsäuredi (2-ethylhexylester) und 101 g (1 mol) Triethylamin.

11.

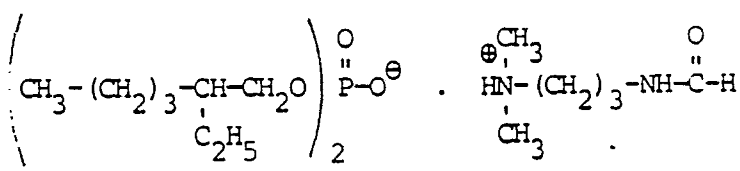

Aus 322 g (1 mol) Phosphorsäuredi (2-ethylhexylester) und 130 g (1 mol) Dimethylaminopropylformamid nach Beispiel 1.

12

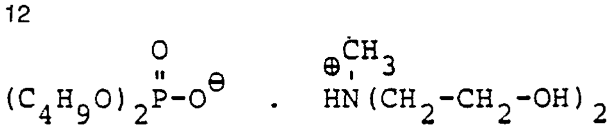

Aus 210 g (mol) Phosphorsäuredibutylester und 119 g (1 mol) Methyldiethanolamin gemäß Beispiel 1, $n_D^{20}=1,4582$.

13.

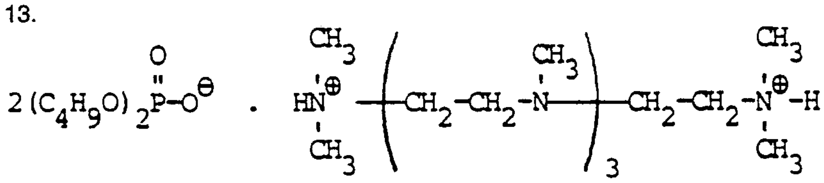

Aus 210 g (1 mol) Phosphorsäuredibutylester und 140 g (0,5 mol) Heptamethyltetraethylenpentamin.

14.

Über 105 g (0,5 mol) Phosphorsäuredibutylester in 100 ml Toluol wird bei 10°C Ammoniak aufgeleitet; der Niederschlag wird abgesaugt, mit Toluol gewaschen und getrocknet. Ausbeute 113 g eines farblosen Pulvers, Schmp. 133-134°C, von glatter Löslichkeit in Wasser.

Analyse für $C_8H_{22}NO_4P$ (227)
ber. N: 6,1 P: 13,6
gef. N: 6,0 P: 13,4

B) Anwendungsbeispiele:

Herstellung von HF-verschweißbaren Weichschaumstoffen mit erhöhtem dielektrischem Verlustfaktor.

Die Herstellung der PU-Weichschaumstoffe erfolgte auf einer kontinuierlich arbeitenden Hochdruck-Blockverschäumungsmaschine (Fa. Hennecke, Birlinghoven, Siegkreis) oder nach dem Handverschäumungsverfahren. Beim Handverschäumungsverfahren werden die Polyole mit Stabilisator, Aktivatoren und Wasser vermischt, nach Zugabe des Isocyanates wird nochmals innig vermischt und das schäumende Gemisch in ein Papierpäckchen gegossen und bei 100°C 30 Minuten nachgeheizt. Je nachdem, ob nach dem Handmisch-Verfahren oder nach dem Maschinenverschäumungs-Verfahren gearbeitet wurde, ist die Rezeptur mit H bzw. M gekennzeichnet. Die flüssigen bzw. durch Zugabe geeigneter Lösungsmittel verflüssigten Ammoniumsalze wurden entweder mit dem Polyol vermischt (Handmischverfahren) oder in den Mischkopf der Schäummaschinen eindosiert.

Zur Überprüfung der HF-Verschweißbarkeit werden zwei je 1 cm starke Schaumstoffplatten bis zur vollständigen Thermoplastifizierung des Schaumstoffes an der Naht verschweißt.

Bei einer Verschweißung von Schaumstoffen mit Textilien wurde eine Sandwich-Konstruktion, bestehend aus 0,8 cm Schaumstoff, unterseitig Polyamid-Charmeuse vom Flächengewicht 60 g/m$^2$, oberseitig Trevira® — Crushed-Velours (Flächengewicht 320 g/m$^2$), eingesetzt.

Als Elektrodenisolation wurde eine Teflon-Glasseide-Unterlage verwendet, die HF-inaktiv ist, so daß die Verschweißbarkeit nur noch von der Thermoplastizität und dem dielektrischen Verlustfaktor des Schaumstoffes abhängt.

Das eingesetzte HF-Schweißgerät war ein Hochfrequenz-Generator vom Typ G 4000 SD der Fa. Kiefel, Freilassing (Leistung 4,3 Kw, Frequenz 27, 12 M Hz).

Folgende Bedingungen wurden eingehalten:

| | **Schaumstoff/Schaumstoff** | **Schaumstoff/-Polyester-Textil** |
|---|---|---|
| Elektrodenfläche: | 1,44x10 cm | 1,44x10 cm |
| Preßkraft: | 250 kp | 250 kp |
| Schweißspannung: | 900 Volt | 1000 Volt |
| Schweißzeit: | variabel | variabel |

Die Verfahrensweise ist im folgenden tabellarisch dargestellt.

TDI 80: Toluylendiisocyanat (80 % 2,4- und 20 % 2,6-Isomeren)
TDI 65: Toluylendiisocyanat (65 % 2,4- und 35 % 2,6-Isomeren)
Polyol:
Gemisch aus
50 % einer nach DE-A 3 008 590 gemäß Beispiel A)5. hergestellten 20 %igen Lösung eines Polyurethans aus Neopentylglykol und TDI 80 in einem linearen Polypropylenglykol-Polyether der OH-Zahl 112 sowie
50 % eines Polyols, gestartet durch Addition von Propylenoxid-Ethylenxoid (90:10) an ein Glycerin-Propylenglykol — 1,2-Gemisch (überwiegend Glycerin) der OH-Zahl 45.

Die Gesamt-OH-Zahl für das Polyolgemisch beträgt 71.

**Tabelle 1:** Herstellung von HF-schweißbaren PU-Weichschaumstoffen unter Zusatz verschiedener Mengen der Verbindung nach Beispiel 3.

| Ausgangskomponenten (Gew-Tle.) | 1.1. (M) | 1.2. (M) | 1.3 (M) | 1.4. (M) |
|---|---|---|---|---|
| Polyol | 100 | 100 | 100 | 100 |
| Wasser | 2,5 | 2,5 | 2,5 | 2,5 |
| handelsüblicher Weichschaumstabilisator (Polyalkylenglykol-Polysiloxan-Copolymeres) | 0,8 | 0,8 | 0,8 | 0,8 |
| Dimethylethanolamin | 0,6 | 0,6 | 0,4 | 0,5 |
| handelsüblicher Aminaktivator (PS 207 der Bayer AG) | 0,15 | 0,15 | 0,15 | 0,15 |
| Zinn-(II)-octoat | 0,15 | 0,1 | 0,1 | 0,08 |
| TDI 80 | 19,5 | 19,5 | % | % |
| TDI 65 | 19,5 | 19,5 | 39,0 | 39,0 |
| TDI-Index | 106 | 106 | 106 | 106 |
| Zusatz nach Beispiel 3 | % | 1 | 2 | 4 |
| **Physikalische Eigenschaften** | | | | |
| Raumgewicht (kg/m$^3$ | 36 | 36 | 35 | 35 |
| Zugfestigkeit (kPa) | 125 | 135 | 140 | 120 |
| Bruchdehnung (%) | 200 | 210 | 280 | 270 |
| Stauchh. bei 40 % (kPa) | 4,2 | 3,9 | 3,5 | 3,2 |
| Druckverformungsrest bei 90 % (%) | 4,8 | 5,2 | 10,3 | 87 |
| Verschweißzeit Schaumstoff/Schaumstoff (sec) | 7 | 5 | 4 | 3 |
| Verschweißzeit Schaumstoff/Polyester-Textil | 10 | 6 | 5 | 4 |

Die entsprechend der in der Tabelle 1 angegebenen Verfahren erhältlichen Schaumstoffe sind offenzellig, und lassen sich störungsfrei mit Textilien und folienartigen Deckschichten problemlos flammkaschieren. Die enstandenen Schweißnähnte sind deutlich ausgeprägt, der Schaumstoff ist zu einem dünnen Film an der Schweißnaht zusammengeschomolzen. Es treten keine Durchbrenner auf und die Schweißnähte lassen sich nicht mehr voneinander trennen, ohne daß der seitliche Schaumstoff einreißt. Bei der Verschweißung von Schaumstoffen mit Polyestertextilien ist eine gut ausgeprägte, feste Naht entstanden. Die Abreißkraft eines 5 cm breiten Streifens (Haftkraft Obermaterial/Schaumstoff) beträgt ca. 60 N. Aus dem Beispiel geht hervor, daß die HF-Schweißbarkeit durch das Zusatzmittel erheblich verbessert wird. Das Resultat ist auch eine erhebliche Verringerung der Schweißzeit und damit eine Verkürzung der Zykluszeiten in Produktionsverfahren auf ein akzeptables Niveau. Es sei daruf hingewiesen, daß die Verschweißzeit Schaumstoff/Schaumstoff üblicherweise mehr als 20 Sekunden beträgt. Ähnlich vorteilhafte Ergebnisse werden erzielt, wenn man erfindungsgemäße Zusätze zu Schaumstoffen gemäß den in Tabelle 2 und 3 charakterisierten Verfahren einsetzt. Entsprechende Ergebnisse sind in den Tabellen 2 und 3 ausführlich wiedergegeben.

**Tabelle 2:** Herstellung von HF-schweißbaren Weichschaumstoffen unter Zusatz diverser Ammoniumsalze der Dibutylphosphorsäure

| Ausgangskomponenten (Gew.-Tle.) | 2.1. (M) | 2.2. (M) | 2.3. (M) | 2.4. (M) | 2.5. (M) | 2.6. (M) | 2.7. (M) | 2.8. (M) |
|---|---|---|---|---|---|---|---|---|
| Polyol | 100 | 100 | 100 | 100 | 100 | 100 | 100 | 100 |
| Wasser | 2,5 | 2,5 | 2,2 | 2,5 | 2,5 | 2,5 | 2,5 | 2,5 |
| handelsüblicher Weichschaumstabilisator (Polyalkylenglykol-Polysiloxan-Copolymeres) | 0,8 | 0,8 | 0,8 | 0,8 | 0,8 | 0,8 | 0,8 | 0,8 |
| Dimethylethanolamin | 0,6 | 0,5 | 0,6 | 0,6 | 0,6 | 0,6 | 0,6 | 0,6 |
| Handelsüblicher Aminaktivator (PS 207 der Bayer AG) | 0,15 | 0,15 | 0,15 | 0,15 | 0,15 | 0,15 | 0,15 | 0,5 |
| Zinn-(II)-oktoat | 0,15 | 0,1 | 0,1 | 0,08 | 0,05 | 0,12 | 0,09 | 0,05 |
| TDI 80 | 19,5 | 9,75 | 19,5 | 9,75 | 19,5 | % | 19,5 | % |
| TDI 65 | 19,5 | 29,5 | 19,5 | 29,25 | 19,5 | 39,0 | 19,5 | 39,0 |
| TDI-Index | 106 | 106 | 106 | 106 | 106 | 106 | 106 | 106 |
| Zusatz gemäß Beispiel 4 | % | 1,5 | % | % | % | % | % | % |
| Zusatz gemäß Beispiel 1 | % | % | 1,5 | % | % | % | % | % |
| Zusatz gemäß Beispiel 13 | % | % | % | 1,5 | % | % | % | |
| Zusatz gemäß Beispiel 5 | % | % | % | % | 1,5 | % | % | % |
| Zusatz gemäß Beispiel 6 | % | % | % | % | % | 1,5 | % | % |
| Zusatz gemäß Beispiel 10 | % | % | % | % | % | % | 1,5 | % |
| Zusatz gemäß Beispiel 14 | % | % | % | % | % | % | % | 1,5 |
| **Physikalische Eigenschaften** | | | | | | | | |
| Raumgewicht (kg/m$^3$) | 36 | 34 | 37 | 36 | 35 | 34 | 34 | 34 |
| Zugfestigkeit (kPa) | 125 | 125 | 145 | 130 | 115 | 140 | 145 | 115 |
| Bruchdehnung (%) | 200 | 266 | 350 | 290 | 250 | 350 | 400 | 380 |
| Stauchh. bei 40 % (kPa) | 4,2 | 2,8 | 3,3 | 2,3 | 2,8 | 2,9 | 2,7 | 2,1 |
| Druckverformungsrest bei 90 % (%) | 4,8 | 8,1 | 7,9 | 11,0 | 14,0 | klebt | 14,0 | klebt |
| Verschweißzeit Schaum/Schaum (sec.) | 7 | 4 | 3,5 | 3,5 | 4 | 3 | 2,5 | 3 |

**Tabelle 3:** Herstellung von HF-schweißbaren Weichschaumstoffen unter Zusatz diverser Ammoniumsalze von Phosphon- und Phosphinsäuren

| Ausgangskomponenten (Gew.-Tle.) | 3.1. (H) | 3.2. (H) | 3.3. (H) |
|---|---|---|---|
| Polyol | 100 | 100 | 100 |
| Wasser | 2,5 | 2,5 | 2,5 |
| handelsüblicher Weichschaumstabilisator (Polyalkylenglykol-Polysiloxan-Copolymeres) | 0,8 | 0,8 | 0,8 |
| Dimethylethanolamin | 0,15 | 1,0 | 0,15 |
| handelsüblicher Aminaktivator (PS 207 der Bayer AG) | 0,15 | 1,5 | 0,6 |
| Zinn-(II)-octoat | 0,5 | 0,15 | 0,1 |
| TDI 80 | 19,5 | 19,5 | 19,5 |
| TDI 65 | 19,5 | 19,5 | 19,5 |
| TDI-Index | 106 | 106 | 106 |
| Zusatz gemäß Beispiel 8 | 1,5 | % | % |
| Zusatz gemäß Beispiel 7 | % | 1,0 | % |
| Zusacz gemäß Beispiel 9 | % | % | 1,5 |
| **Physikalische Eigenschaften** | | | |
| Raumgewicht (kg/m$^3$ | 34 | 35 | 35 |
| Zugfestigkeit (kPa) | 110 | 70 | 80 |
| Bruchdehnung (%) | 400 | 200 | 360 |
| Stauchh. bei 40 % (kPa) | 1,9 | 1,5 | 2,0 |
| Druckverformungsrest bei 90 % (%) | klebt | klebt | 88 |
| Verschweißzeit Schaum/Schaum (sec) | 3 | 5 | 2,5 |

## Patentansprüche

1. Verfahren zur Herstellung von offenzelligen, hochfrequenzverschweißbaren Polyurethanschaumstoffen durch U-setzung von mindestens 2 gegenüber Isocyanaten reaktionsfähige Wasserstoffatome aufweisenden höhermolekularen Verbindungen vom Molekulargewicht 400 bis 10 000 mit Polyisocyanaten und gegebenenfalls Kettenverlängerungsmitteln vom Molekulargewicht 18 bis 399 in Gegenwart von Katalysatoren, gegebenenfalls

Schaumstabilisatoren, Wasser und/oder organischen Treibmittelzusätzen und üblichen Hilfs- und Zusatzstoffen, dadurch gekennzeichnet, daß man Ammoniumsalze phosphorhaltiger Säuren einsetzt, die glatt in Polyolen löslich sind und Phosphorsäure-mono- und -dialkylester, Dialkyl-phosphinsäuren, Alkylphosphonsäuren bzw. ihre Monoalkylester als phosphorhaltige Säuren enthalten, und sie den Schaumstoff-Ausgangskomponenten, gelöst in einer oder mehreren der Ausgangskomponenten, in Mengen von 0,1 bis 10 Gew.-%, bezogen auf die Gesamtmischung, zusetzt.

2. Verfahren nach Anspruch 1, dadurch gekennzeichnet, daß man als Ammoniumsalze phosphorhaltige Säuren Salze von Phosphorsäuredibutylester, Phosphorsäure-bis-(2-ethyl-hexylester), Dibutylphosphinsäure, Methylphosphonsäure oder Methylphosphonsäuremonomethylester mit basischen Stickstoff enthaltenden Verbindungen aus der Reihe Dimethylaminoethylformamid, Dimethylaminopropylformamid, Bis dimethylaminopropyl)-formamid, Ammoniak, N-Methyl-, N'-formyl-piperazin, Iris-(dimethylaminopropyl)-amin, Tetramethyl-hexamethylendiamin, Heptamethyltetraethylenpentamin, Pentamethyl-diethylentriamin, Dimethylaminopropyl-morpholin, N-Formyl-N'-bis(dimethylaminopropyl)-propan-1,3-diamin, Formylaminopropylmorpholin, N-Methyl-N'-formylaminopropyl-piperazin, Ethanolamin, Die erhaltenen Schaumstoffe entsprechend Tabelle 2 und 3 sind gleichfalls offenzellig, störungsfrei sowie mit textilen bzw. folienartigen Deckschichten problemlos HF-schmelzkaschierbar. Die entstandenen Schweißnähte sind deutlich ausgeprägt, der Schaumstoff ist zu einem dünnen Film zusammengeschmolzen. Es treten keine Durchbrenner auf und die Schweißnähte lassen sich nicht mehr voneinander trennen, ohne daß der seitliche Schaum reißt.

Diethanolamin, Dimethylethanolamin, Methyldiethanolamin, Triethylamin, Dimethylcyclohexylamin, Piperazin, Triethylendiamin, Diisopropylamin, Hexamethylendiamin, Bis-(2-hydroxethyl)-amino-methylphosphonsäuredimethylester, Adipinsäure-bis-(2-dimethylaminoethylester), 1,8-Diazabicyclo-(5,4,0)-undec-7-en, 1,5-Diazabicyclo-(5,4,0)-non-5-en, Orthoameisensäure-tris-dimethyl-aminoethylester, Bis-(dimethylaminoethyl)-ether zusetzt.

3. Verfahren nach Ansprüchen 1 und 2, dadurch gekennzeichnet, daß man Ammoniumsalze der Dibetylphosphorsäure verwendet.

4. Verfahren nach Ansprüchen 1 bis 3, dadurch gekennzeichnet, daß man als Ammoniumsalze

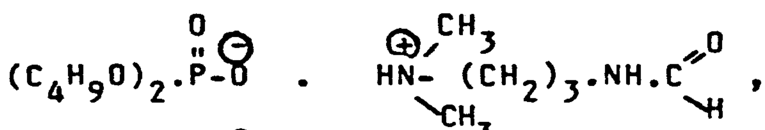
,

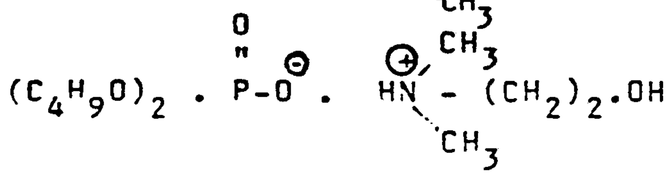

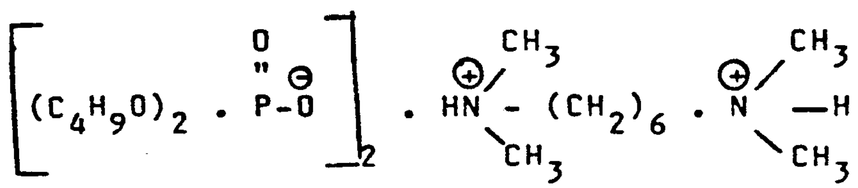

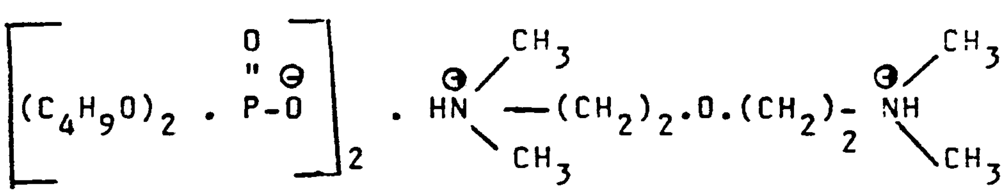

oder

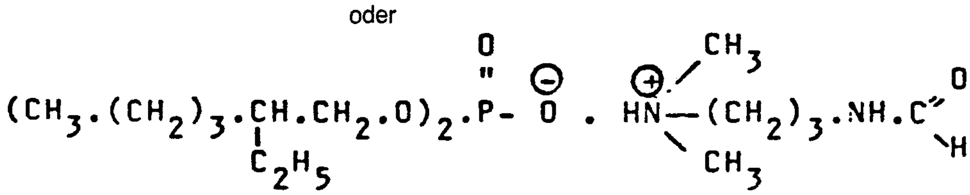

zusetzt.

5. Verfahren nach Ansprüchen 1 und 2, dadurch gekennzeichnet, daß man als Ammoniumsalze

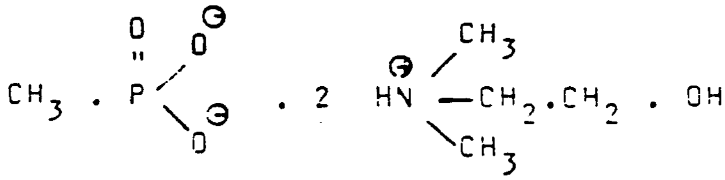

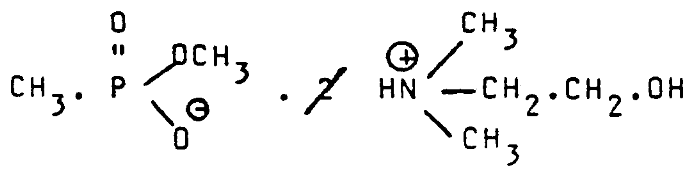

zusetzt.

6. Verfahren nach Anspruch 1, dadurch gekennzeichnet, daß man Ammoniumsalze phosphorhaltiger Säuren entsprechend der Formel

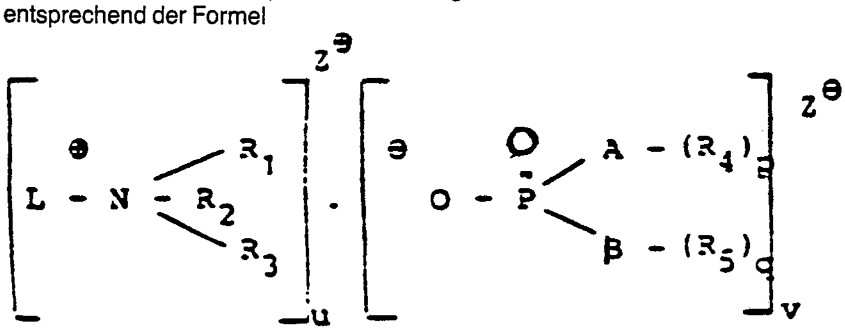

[Kation] [Anion]

wobei (es gilt: $u \cdot z^{\ominus} = v \cdot z^{\ominus}$)

I. für den kationischen Aminteil

L Wasserstoff und/oder Alkyl von $C_1$ bis $C_6$

$R_{1,2,3}$ $-(CH_2)_e-T$, wobei

e eine ganze Zahl von 1 bis 10 und

T Wasserstoff, Hydroxy, 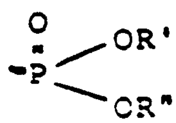 und 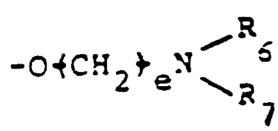

R',R" gleichen oder verscheidenen Alkylresten von $C_1$ bis $C_{10}$, gegebenenfalls auch durch Hydroxy substituiert, oder

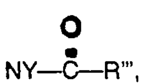, mit

R' " gleich Wasserstoff oder ein Alkylrest von $C_1$ bis $C_6$:

Y Wasserstoff oder $-(CH_2)_e-T$ sein kann,

und/oder

$R_{1,2,3}$ 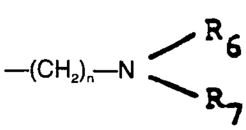 wobei

$R_6$, $R_7$ gleiche oder verschiedene, lineare oder verzweigte Alkylreste von $C_1$ bis $C_6$.

n eine ganze Zahl von 2 bis 10

und/oder

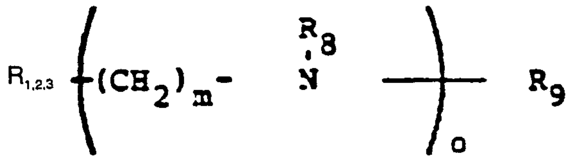

wobei

$R_8$, $R_9$ gleiche oder verschiedene, lineare oder verzweigte Alkylreste von $C_1$ bis $C_6$

m eine ganze Zahl von 2 bis 10.

o eine ganze Zahl von 2 bis 6 sein kann und

$z^{\oplus}$ die Ladungszahl des Kations ist und der Gesamtzahl an N-Atomen entspricht oder kleiner als diese sein kann,

u je nach Ladungszahl $Z^{\oplus}$ des Kationenrestes diser u-mal enthalten sein kann. wobei gilt, daß $u \cdot Z^{\oplus} = v \cdot Z^{\ominus}$ ist

II. sowie für das phosphorhaltige Anion

A, $—CH_2—$ oder —O—

B, $—CH_2—$

$z^{\ominus}$ die Ladungszahl des Anions ist,

v je nach Ladungszahl $Z^{\ominus}$ des Anions dieser anionische Rest v-mal enthalten ist, und eine ganze Zahl zwischen 1 und 3 bedeutet, wofür gilt, daß $v \cdot Z^{\ominus} = u \cdot Z^{\oplus}$ ist,)

$R_4$, $R_5$ Wassterstoff und/oder einen $C_1$—$C_{10}$—Alkyl- oder -Cycloalkylrest.

den Schaumstoff-Ausgangskomponenten, gelöst in einer oder mehreren der Ausgangskomponenten, in Mengen von 0,1 bis 10 Gew.-%, bezogen auf die Gesamtmischung, zusetzt.

7. Hochfrequenzverschqeißbare, offenzellige Polyurethanschaumstoffe aus höhermolekularen Polyhydroxylverbindungen vom Molekulargewicht 400 bis 10 000, Polyisocyanaten, gegebenenfalls Kettenverlängerungsmitteln Molekulargewicht 18 bis 399, gegebenenfalls Katalysatoren, Schaumstabilisatoren, Wasser und/oder organischen Treibmittelzusätzen, dadurch gekennzeichnet, daß sie 0,1—10 Gew.-% Ammoniumsalze phosphorhaltiger Säuren enthalten, wobei die phosphorhaltigen Säuren Phosphorsäure-mono- und -dialkylester, Dialkylphosphinsäuren, Alkylphosphonsäuren bzw. ihre Monoalkylester darstellen.

8. Hochfrequenzverschweißbare, offnzellige Polyurethanschaumstoffe nach Anspruch 7, dadurch gekennzeichnet, daß sie 0,1 bis 10 Gew.-% Ammoniumsalze phosphorhaltiger Säuren enthalten, wobei die zur Ammoniumsalzbildung herangezogenen Amine. Dimethylaminoethylformadid, Dimethylaminopropylformamid, Bis(dimethylaminopropyl)-formamid, Ammoniak, N-Methyl-, N'-formyl-piperazin, Tris-(dimethylaminopropyl)-amin, Tetramethyl-hexamethylendiamin, Heptamethyltetraethylenpentamin, Pentamethyl-diethylentriamin, Dimethylaminopropyl-morpholin, N-Formel-N'-bis(dimethylaminopropyl)-propan-1,3-diamin, Formylaminopropylmorpholin, N-Methyl-N'-formylaminopropyl-piperazin, Ethanolamin.

Diethanolamin, Dimethylethanolamin, Methyldiethanolamin, Triethylamin, Dimethylcyclohexylamin, Piperazin, Triethylendiamin, Diisopropylamin, Hexamethylendiamin, Bis-(2-hydroxyethyl)-amino-methylphosphonsäuredimethylester, Adipinsäure-bis-(2-dimethylaminoethylester), 1,8-Diazabicyclo-(5,4,0)-undec-7-en, 1,5-Diazabicyclo-(5,4,0)-non-5-en, Orthoameisensäure-tris-dimethylaminoethylester, Bis-(dimethylaminoethyl)-ether sind.

9. Hochfrequenzverschweißbare, offenzellige Polyurethanschaumstoffe nach Anspruch 7, dadurch gekennzeichnet, daß sie

0,1 bis 10 Gew.-% an Ammoniumsalzen

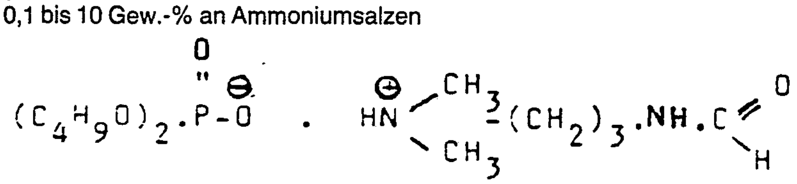

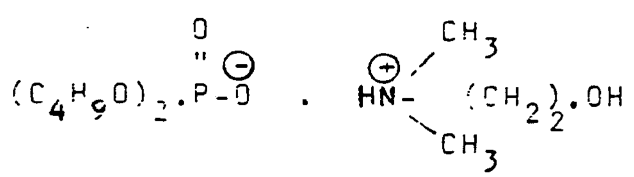

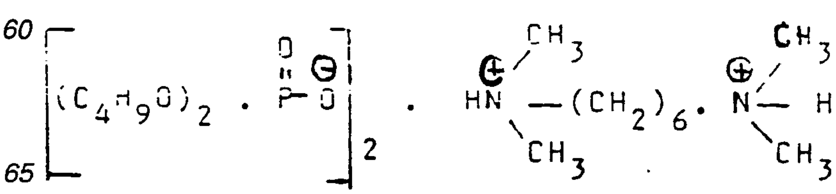

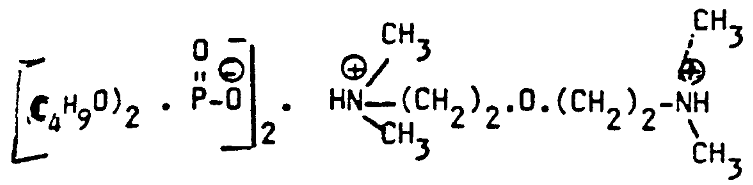

oder

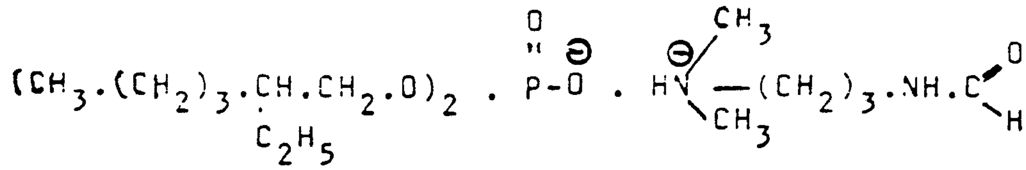

enthalten.

10. Hochfrequenz verschweißbare, offenzellige Polyurethanschaumstoffe nach Anspruch 7, dadurch gekennzeichnet, daß sie 0,1 bis 10 Gew.-% Ammoniumsalze phosphorhaltiger Säuren der Formel

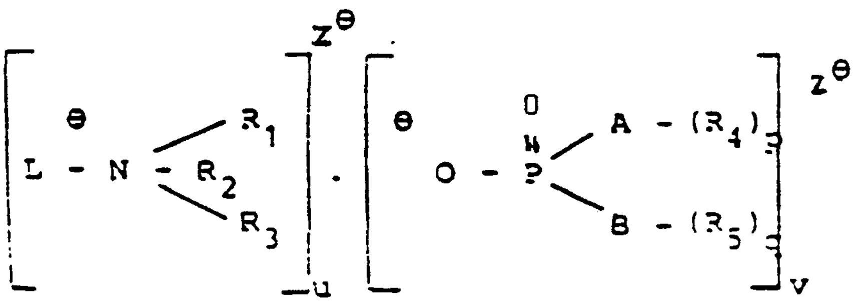

[Kation] · [Anion]
(es gilt: $u \cdot Z^{\ominus} = v \cdot Z^{\ominus}$)

enthalten, wobei die Substituenten am Stickstoff und Phosphor die in Anspruch 6 angegebene Bedeutung haben.

**Claims**

1. Process for the production of open-celled, high-frequency weldable polyurethane foams by reacting relatively high molecular weight compounds having a molecular weight of 400 to 10 000 and containing at least 2 hydrogen atoms which are reactive towards isocyanates with polyisocyanates and optionally chain-lengthening agents having a molecular weight of 18 to 399, in the presence of catalysts, optionally foam stabilisers, water and/or organic blowing agent additives and customary auxiliaries and additives, characterised in that ammonium salts of phosphorus-containing acids are used which are readily soluble in polyols and contain phosphoric acid mono- and dialkyl esters, dialkyl phosphinic acids, alkyl phosphonic acids or monoalkyl esters thereof as the phosphorus-containing acids, and they are added to the foam starting components, dissolevd in one or more of the starting components, in quantities of 0.1 to 10 % by weight, based on the total mixture.

2. Process according to Claim 1, characterised in that the ammonium salts of phosphorus-containing acids added are salts of phosphoric acid dibutyl ester, phosphoric acid bis-(2-ethyl-hexylester), dibutyl phosphinic acid, methyl phosphonic acid or methyl phosphonic acid monomethyl ester with basic nitrogen-containing compounds from the series comprising dimethylaminoethyl formamide, dimethylaminopropyl formamide, bis(dimethylaminopropyl)-formamide, ammonia, N-methyl-, N'-formyl-piperazine, tris-(dimethylaminopropyl)-amine, tetramethyl-hexamethylene diamine, heptamethyltetraethylenepentamine, pentamethyl-diethylenetriamine, dimethylaminopropyl-morphline, N-formyl-N'-bis (dimethylaminopropyl)-propane-1,3-diamine, formylaminopropyl-morpholine, N-methyl-N'-formylaminopropyl-piperazine, ethanolamine, diethanolamine, dimethyl ethanolamine, methyl diethanolamine triethylamine, dimethyl cyclohexylamine, piperazine, triethylene diamine, diisopropylamine, hexamethylene diamine, bis-(2-hydroxyethyl)-amino-methyl phosphonic acid dimethyl ester, adipic acid bis-(2-dimethylaminoethyl ester), 1,8-diazabiocyclo-(5,4,0)-undec-7-ene, 1,5-diazabicyclo-(5,4,0-non-5-ene, orthoformic acid tris-dimethylaminoethyl ester or bis-(dimethylaminoethyl) ether.

3. Process according to Claims 1 and 2, characterised in that ammonium salts of dibutylphosphoric acid are used.

4. Process according to Claims 1 to 3, characterised in that

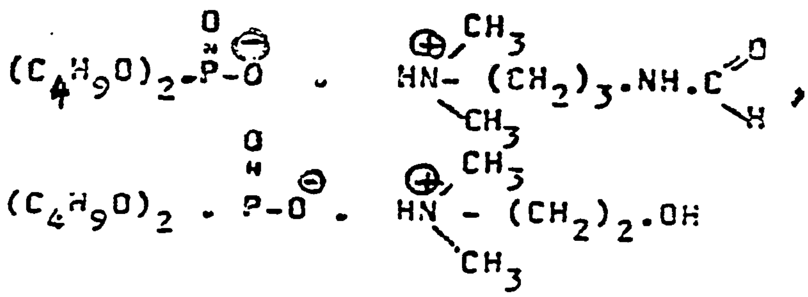

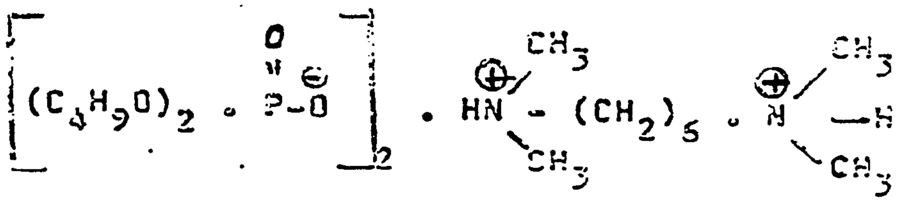

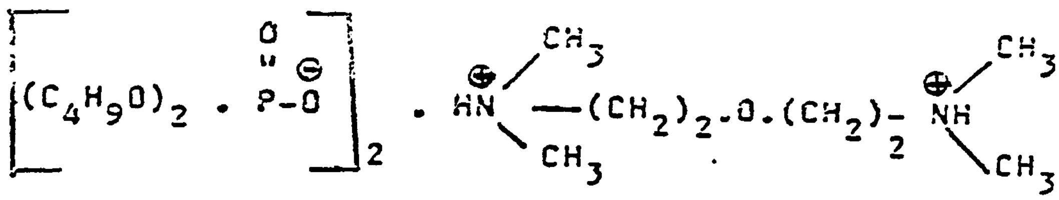

or

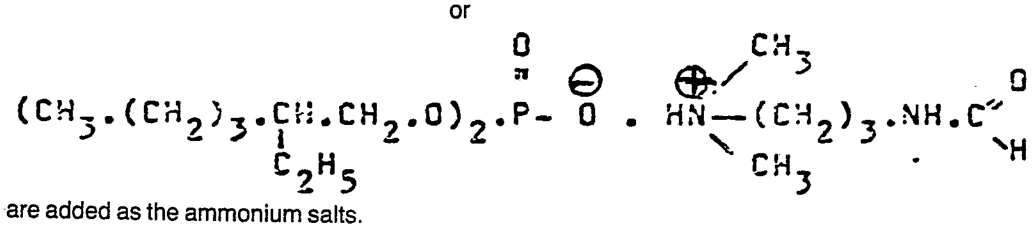

are added as the ammonium salts.

5. Process according to Claims 1 and 2, characterised in that

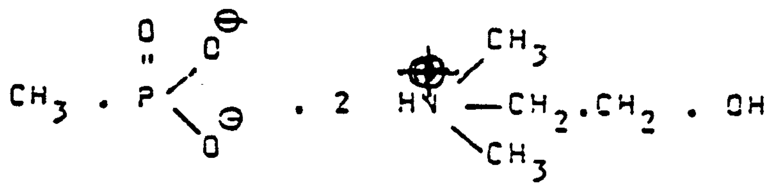

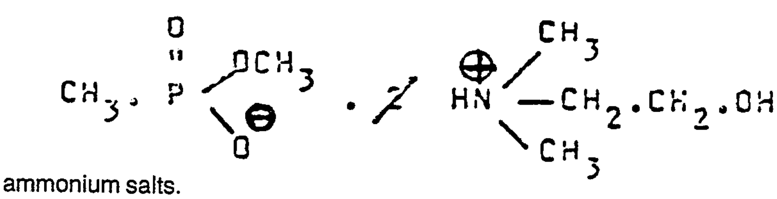

are added as the ammonium salts.

6. Process according to Claim 1, characterised in that ammonium salts of phosphorus-containing acids corresponding to the formula

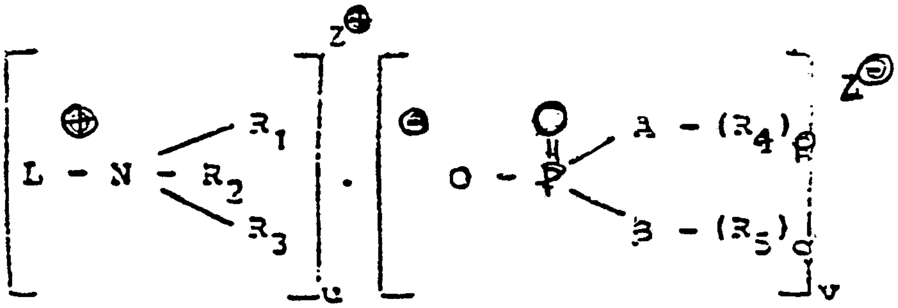

[cation] [anion]
(the following applies: $u \cdot Z^{\oplus} = V \cdot Z^{\ominus}$)

wherein

I. in the case of the cationic amine portion

| | |
|---|---|
| L | is hydrogen and/or alkyl with $C_1$ to $C_6$ |
| $R_{1,2,3}$ | is $-(CH_2)_e-T$, wherein e is an integer from 1 to 10 and |

T is hydrogen, hydroxyl, 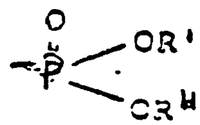 and $-O-(CH_2)-_eN$ 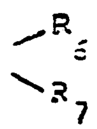

and

| | |
|---|---|
| R' and R" | are identical or different alkyl radicals with $C_1$ to $C_{10}$, optionally sibstituted by hydroxyl, or 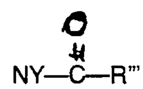 in which |
| R''' | is hydrogen or an alkyl radical with $C_1$ to $C_6$; |
| Y | can be hydrogen or $-(CH_2)_e-T$ and/or |
| $R_{1,2,3}$ | is $-(CH_2)_n-N$ 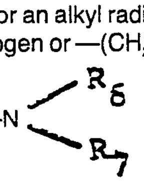 wherein |
| $R_6$ and $R_7$ | are identical or different, linear or branched alkyl radicals with $C_2$ to $C_6$. |
| n | is an integer from 2 to 10 and/or |

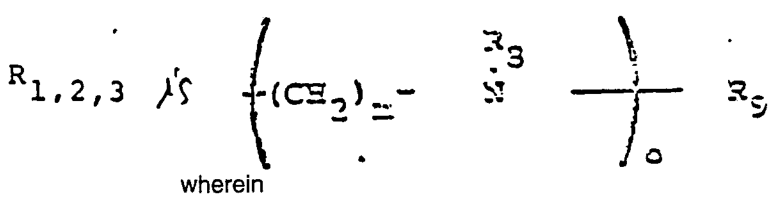

wherein

| | |
|---|---|
| $R_8$ and $R_9$ | are identical or different, linear or branched alkyl radicals with $C_1$ to $C_6$, |
| m | is an integer from 2 to 10, |
| o | can be an integer from 2 to 6 and |
| $Z^{\oplus}$ | is the charge number of the cation and corresponds to the total number of N atoms or can be smaller than the letter, |
| u | depending on the charge number $Z^{\oplus}$ of the cationic radical the latter can be present u times, such that $u \cdot Z^{\oplus} = v \cdot Z^{\ominus}$, |

II. and in the case of the phosphorus-containing anion

| | |
|---|---|
| A | is $-CH_2$ or $-O-$ |
| B | is $-CH_2-$ |
| $Z^{\ominus}$ | is the charge number of the anion, |
| v | depending on the charger number $Z^{\ominus}$ of the anion, this anionic radical is present v times, and deontes an integer between 1 and 3, such that $v \cdot Z^{\ominus} = u \cdot Z^{\oplus}$, |

$R_4$ and $R_5$ are hydrogen and/or a $C_1$—$C_{10}$—alkyl or -cycloalkyl radical are added to the foam starting components, dissolved in one or more of the starting components, in quantities of 1.0 to 10 % by weight, based on the total mixture.

7. High-frequency weldable, open-celled polyurethane foams obtained from relatively high molecular weight polyhydroxyl compounds having a molecular weight of 400 to 10,000, polyisocyanates, optionally chain-lengthening agents molecular weight of 18 to 399, optionally catalysts, foam stabilisers, waters and/or organic blowing agent additives, characterised in that they contain 0.1 — 10 % by weight of ammonium salts of phosphorus-containing acids, the phosphorus-containing acids being phosphoric acid mono- and dialkyl esters, dialkyl phosphinic acids, alkyl phosphonic acids or their monoalkyl esters.

8. High-frequency weldable, open-celled polyurethane foams according to Claim 7, characterised in that they contain 0.1 to 10 % by weight of ammonium salts of phosphorus-containing acids, the amines used for the formation of the ammonium salts being dimethylaminoethyl formamide, dimethylaminopropyl formamide, bis(dimethylaminopropyl) formamide, ammonia, N-methyl-, N'-formyl-piperazine, tris-(dimethylaminopropyl)-amine, tetramethyl-hexamethylene diamine, heptamethyl-tetraethylene pentamine, pentamethyl-diethylene triamine, dimethylaminopropyl-morpholine, N-formyl-N'-bis(dimethylaminopropyl)-propane-1,3-diamine, formylaminopropyl-morpholine, N-methyl-N'-formylaminopropyl-piperazine, ethanolamine, diethanolamine, dimethyl ethanolamine, methyl diethanolamine, triethylamine, dimethyl cyclohexylamine, piperazine, triethylene diamine, diisopropylamine, hexamethylene diamine, bis-(2-hydroxyethyl)-amino-methyl phosphonic acid dimethyl ester, adipic acid bis-(2-dimethylaminoethyl ester), 1,8-diazabicyclo-(5,4,0)-undec-7-ene, 1,5-diazabicyclo-(5,4,0)-non-5-ene, orthoformic acid tris-dimethylaminoethyl ester or bis-(dimethylaminoethyl) ether.

9. High-frequency weldable, open-celled polyurethane foams according to Claim 7, characterised in that they contain 0.1 to 10 % by weight of ammonium salts

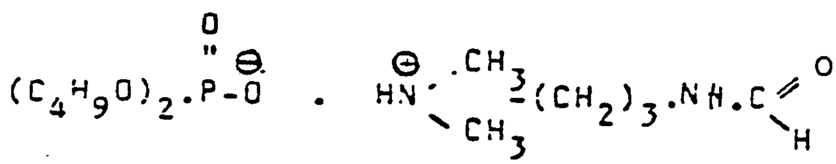

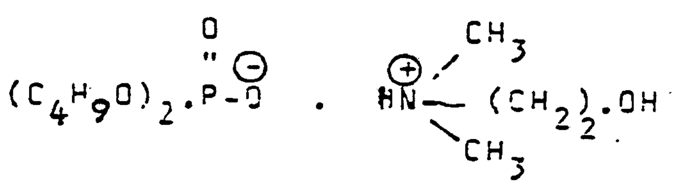

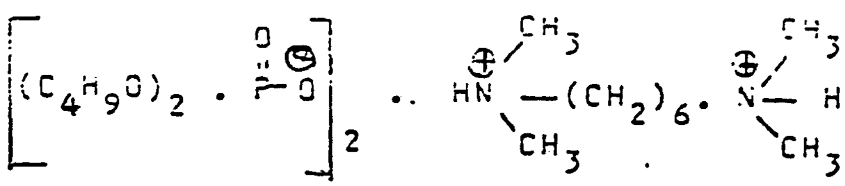

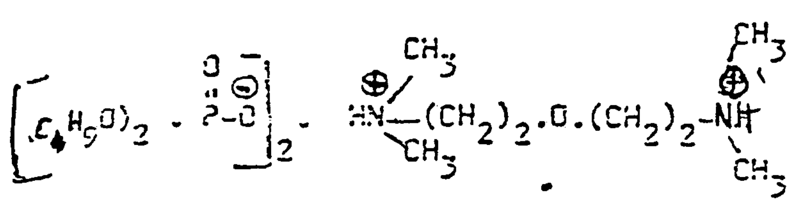

or

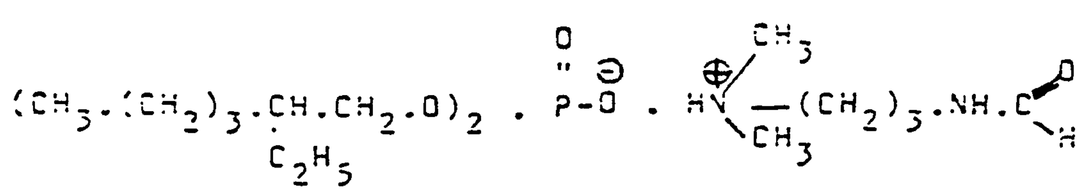

10. High-frequency weldable open-called polyurethane foams according to Claim 7, characterised in that they contain 0.1 to 10 % by weight of ammonium salts of phosphorus-containing acids of the formula

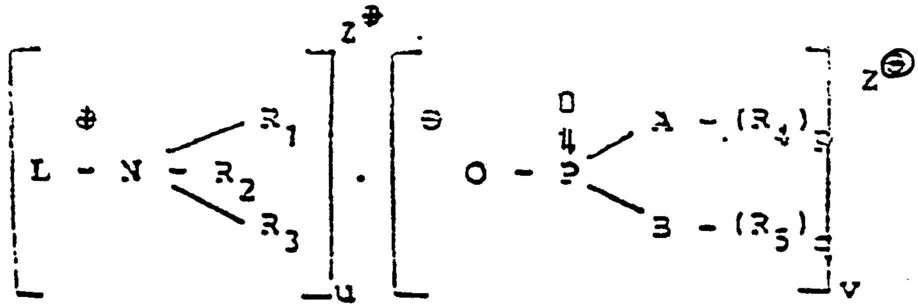

[cation] [anion]

(the following applies: $u \cdot Z^{\oplus} = v \cdot Z^{\ominus}$)

the substituents on the nitrogen and phosphorus having the meaning given in Claim 6.

**Revendications**

1. Procédé de production de mousses de polyuréthanne à cellules ouvertes, soudables par heute fréquence, par réaction de composés de poids moléculaire élevé présentant au moins 2 atomes d'hydrogène aptes à réagir vis-à-vis d'isocyanates et ayant un poids moléculaire de 400 à 10 000 avec des polyisocyanates et, le cas échéant, des agents d'allongement de chaîne d'un poids moléculaire de 18 à 399, en présence de catalyseurs, éventuellement d'agents de stabilisation de mousse, d'eau et/ou d'additifs organiques porogènes et d'adjuvants et d'additifs classiques, caractérisé en ce qu'on utilise des sels d'ammonium d'acides contenant du phosphore qui se dissolvent bien dans des polyols et qui comportent comme acides contenant du phosphore, des esters monoalkyliques et dialkyliques d'acide phosphorique, des acides dialkylphosphiniques, des acides alkylphosphoniques et leurs esters monoalkyliques, et on les ajoute aux composants de départ de la mousse, dissous dans un ou plusieurs des composants de départ, en quantités de 0,1 à 10 % en poids par rapport au mélange total.

2. Procédé suivant la revendication 1, caractérisé en ce qu'on ajoute comme sels d'ammonium d'acides contenant du phosphore, des sels de l'ester dibutylique d'acide phosphorique, de l'ester de bis-(2-éthylhexyle) de l'acide phosphorique, d'acide dibutylphosphinique, d'acide méthylphosphonique ou de l'ester monométhylique de l'acide méthylphosphonique avec des composés basiques contenant de l'azote, de la série diméthylaminoéthylformamide, diméthylaminopropylformamide, bis-(diméthylaminopropyl)-formamide, ammoniac, N-méthyl-, N'-formylpiérazine, tris-(diméthylaminopropyl)-amine, tétraméthylhexaméthylènediamine, heptaméthyl-tétraéthylènepentamine, pentaméthyldiéthylènetramine, diméthylaminopropulmorpholine, N-formyl-N'-bis(diméthylaminopropyl)-propane-1,3-diamine, formylaminopropylmorpholine, N-méthyl-N'-formylaminopropylpipérazine, éthanolamine, diéthanolamine, diméthyléthanolamine, méthyldiéthanolamine, triéthylamine, diméthylcyclohexylamine, pipérazine, triéthylènediamine, diisopropylamine, hexaméthylènediamine, ester diméthylique d'acide bis-(2-hydroxyéthyl)-aminométhylphosphonique, ester bis-(2-diméthylaminoéthylique) d'acide adipique, 1,8-diazabicyclo-(5,4,0)-undec-7-ène, 1,5-diazabicyclo-(5,4,0)-non-5-ène, ester tris-diméthylaminoéthylique d'acide orthoformique, éther de bis-(diméthylaminoéthyle).

3. Procédé suivant les revendications 1 et 2, caractérisé en ce qu'on utilise des sels d'ammonium de l'acide dibutylphosphorique.

4. Procéde suivant les revendications 1 à 3, caractérisé en ce qu'on ajoute comme sels d'ammonium

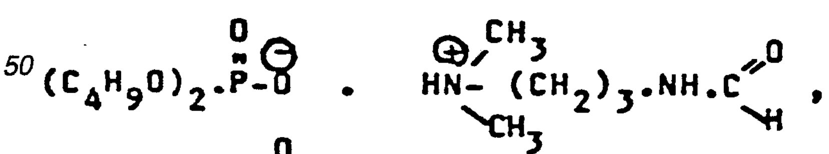

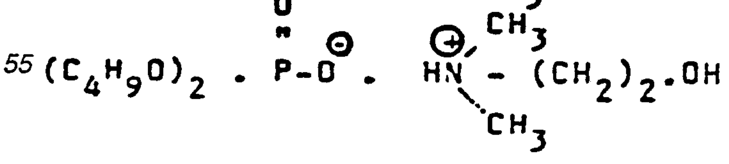

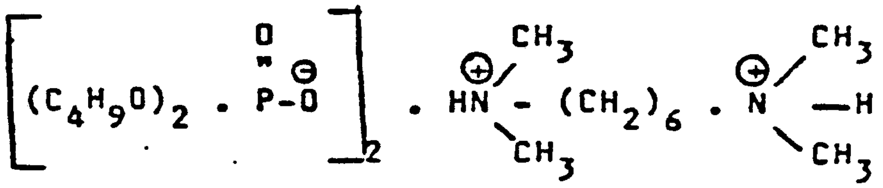

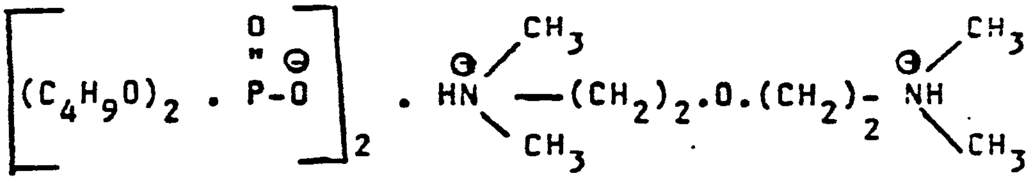

ou

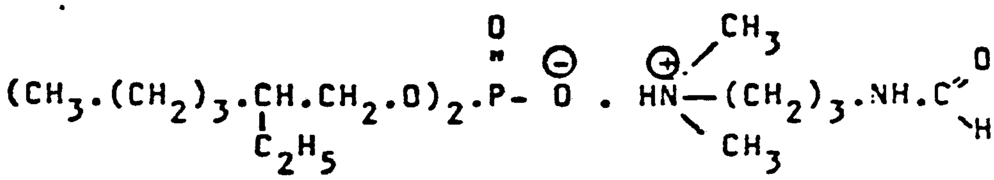

5. Procédé suivant les revendications 1 et 2, caractérisé en ce qu'on ajoute comme sels d'ammonium

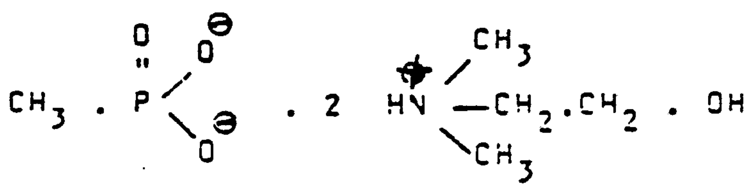

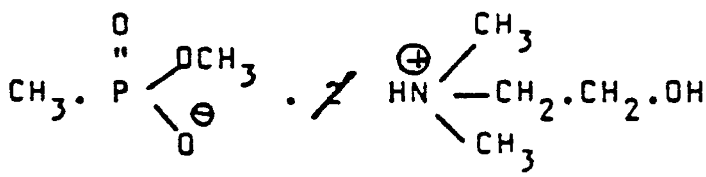

6. Procéde suivant la revèndication 1, caractérisé en ce qu'on ajoute aux composants de départ de la mousse, dissous dans un ou plusieurs das composants de départ en quantités de 0,1 à 10 % en poids par rapport au mélange total, des sels d'ammonium d'acides contenant du phosphore répondant à la formule

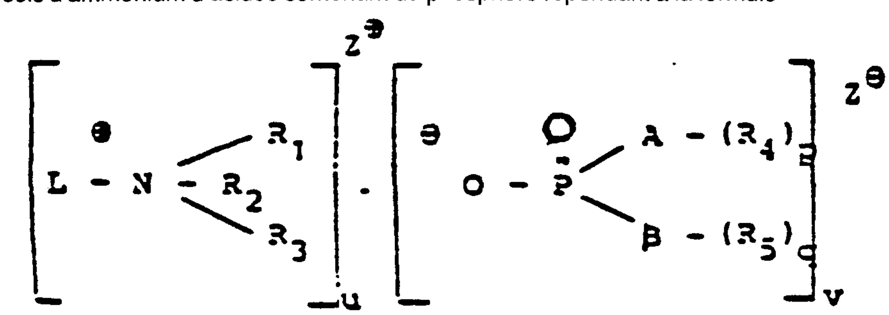

[cation] [Anion]
(on a : $u \cdot Z^{\oplus} = v \cdot Z^{\ominus}$)

ou

I. pour la partie aminée cationique,

L désigne l'hydrogène ou un groupe alkyle en $C_1$ à $C_6$

$R_{1,2,3}$ représentent un groupe $-CH_2)_e-T$, ou

e est un nombre entier de 1 à 10 et

T désigne l'hydrogène et des groupes hydroxy, 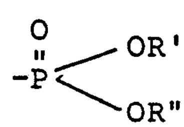

et —O—$(CH_2)_e$N 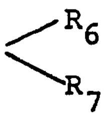

et

R', R" sont des restes alkyle identiques ou différents en $C_1$ à $C_{10}$, éventuellement substitués aussi par un radical hydroxy, ou un groupe NY—$\overset{O}{\overset{\|}{C}}$—R''' dans lequel

R''' représente l'hydrogène ou un reste alkyle en $C_1$ à $C_6$;

Y peut être l'hydrogène ou un groupe —$(CH_2)_e$—T

et/ou

$R_{1,2,3}$ représentent un groupe —$(CH_2)_n$—N 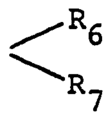 dans lequel

$R_6$, $R_7$ sont des restes alkyle linéaires ou ramifiés identiques ou différents en $C_1$ à $C_6$.

n est un nombre entier de 2 à 10

et/ou

$R_{1,2,3}$ représentent un groupe 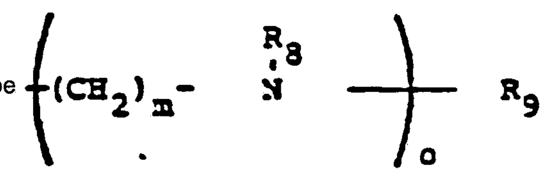

dans lequel

$R_8$, $R_9$ sont des restes alkyle linéaires ou ramifiés identiques ou différents en $C_1$ à $C_6$

m est un nombre entier de 2 à 10

g peut être un nombre entier de 2 à 6 et

$Z^{\oplus}$ désigne le nombre de charges du cation et correspond au nombre total d'atomes d'azote ou peut être inférieur à ce nombre,

u selon le nombre de cbarges $Z^{\oplus}$ du reste cationique, celui-ci peut être contenu u fois, et on a alors $u \cdot Z^{\oplus} = v \cdot Z^{\ominus}$

II. De même pour l'anion contentant du phosphore,

A représente —$CH_2$— ou —O—

B représente —$CH_2$—

$Z^{\ominus}$ désigne le nombre de charges de l'anion,

v selon le nombre de charges $Z^{\ominus}$ de l'anion, ce reste anionique est contenu v fois et il représente un nombre entier de 1 à 3, et on a alors l'équation $v \cdot Z^{\ominus} = u \cdot Z^{\oplus}$,

$R_4$, $R_5$ représentent de l'hydrogène et/ou reste alkyle ou cycloalkyle en $C_1$ à $C_{10}$.

7. Mousses de polyuréthanne à cellules ouvertes soudables par haute fréquence, obteneus à partir de composés polyhydrozyliques de poids moléculaire élevé, d'un poids moléculaire de 400 à 10 000, de polyisocyanates, le cas échéant d'agents d'allongement de chaîne d'un poids moléculaire de 18 à 399, éventuellement de catalyseurs, d'agents stabilisants pour mousse, d'eau et/ou d'additifs organiques porogènes, caractérisées en ce qu'elles contiennent 0,1 à 10 % en poids de sels d'ammonium d'acides contenant du phosphore, les acides contenant du phosphore représentant des esters monoalkyliques et dialkyliques d'acide phosphorique, des acides dialkylphosphiniques, des acides alkylphosphoniques ou leurs esters monoalkyliques.

8. Mousses de polyréthanne à cellules ouvertes, soudables par haute fréquence, suivant la revendication 7, caractérisées en ce qu'elles contiennent 0,1 à 10 % en poids de sels d'ammonium d'acides contenant du phosphore, les amines utilisées pour la formation de sels d'ammonium étant le diméthylaminoéthylformamide, le diméthylaminopropylformamide, le bis (diméthylaminopropyl) — formamide, l'ammoniac, la N-méthyl-, N'-formylpipérazine, la tris-(diméthylaminopropyl)-amine, la tétraméthylhexaméthylènediamine, l'heptaméthyltétraéthylènepentamine, la pentaméthyldiéthylènetriamine, la diméthylaminopropylmorpholine, la N-formyl-N'-bis(diméthylaminopropyl)-propane-1,3-diamine, la formylaminopropylmorpholine, la N-methyl-N'-formylaminopropylpipérazine, l'éthanolamine, la diéthanolamine, la diméthyléthanolamine, la méthyldiéthanolamine, la triéthylamine, la diméthylcyclohexylamine, la pipérazine, la triéthylènediamine, la diisopropylamine, l'hexaméthylènediamine, l'ester diméthylique de l'acide bis-(2-hydroxyéthyl)-aminométhylphosphonique, l'ester bis-(2-diméthylaminoéthylique de l'acide adipique, le 1,8-diazabicyclo-(5,4,0)-undec-7-ène, le 1,5-diazabicyclo-(5,4,0)-non-5-ène, l'ester tris-diméthylaminoéthylique de l'acide orthoformique, l'éther de bis-(diméthylaminoéthyle).

9. Mousses de polyuréthanne à cellules ouvertes, soudables par haute fréquence, suivant la revendication 7, caractérisées en ce qu'elles contienent 0,1 à 10 % en poids de sels d'ammonium

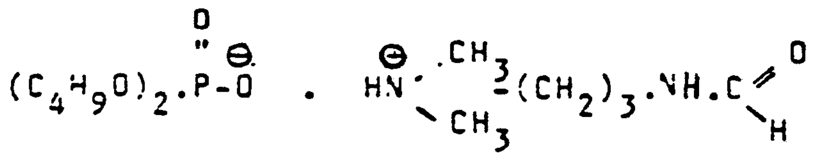

,

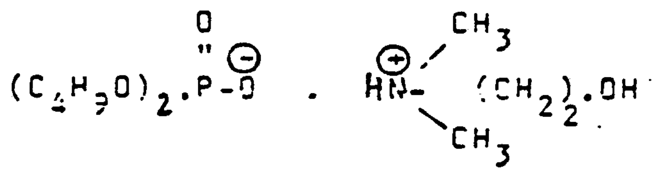

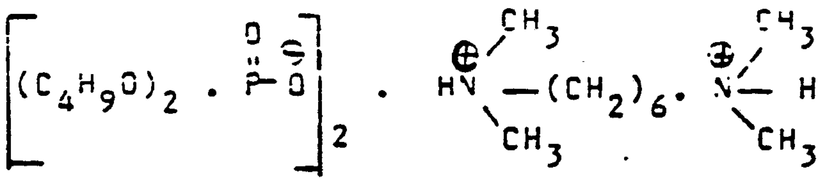

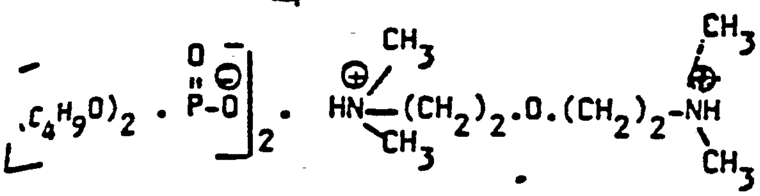

ou

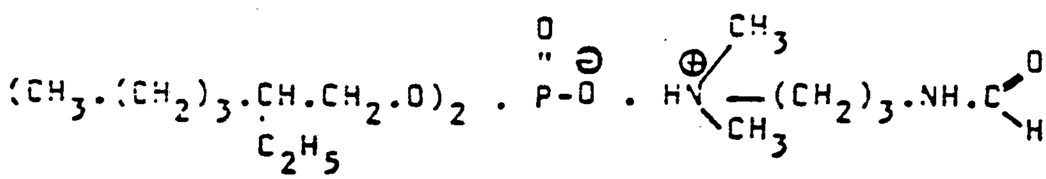

10. Mousses de polyuréthanne à cellules ouvertes soudables par haute fréquence suivant la revendication 7, caractérisées en ce qu'elles contienent 0,1 à 10 % en poids de sels d'ammonium d'acides contenant du phosphore de formule

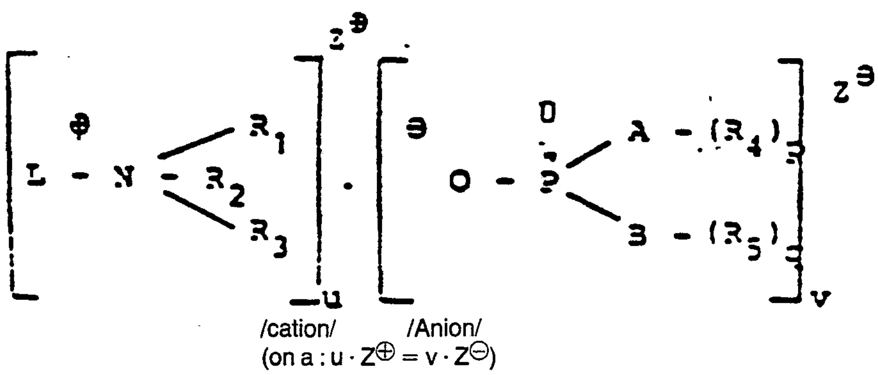

/cation/ /Anion/
(on a : $u \cdot Z^{\oplus} = v \cdot Z^{\ominus}$)

les substituants de l'azote et du phosphore ayant la définition indiquée dans la revendication 6.